# EUROPEAN PATENT APPLICATION

(11) **EP 1 088 830 A2**
(43) Date of publication of application: **04.04.2001**
(21) Application number: 00121167.1
(22) Date of filing: 22.06.1988
(51) Int. Cl.: C07K 14/02, A61K 39/29, C12N 15/40, A61P 31/14

(54) **Hepatitis b surface antigen particles**

(30) Priority: 22.06.1987 EP 87108914; 22.06.1987 EP 87108915
(62) Divisional of application: 88109946.9
(71) Applicant: MEDEVA HOLDINGS B.V., 1078 ED Amsterdam (NL)
(72) Inventor: Thoma, Hans A., 80802 München (DE)
(74) Representative: Campbell, Patrick John Henry

(57) **Abstract**

The invention provides an immunogenic particle comprising a plurality of copies of a first peptide monomer, said peptide monomer comprising a first discrete region and a second discrete region, said first region comprising an HBV pre-S₁ epitope and said second region comprising a peptide which upon secretion will direct formation of particles that will provoke an immune response against the pre-S₁ epitope, wherein said first peptide monomer constitutes more than five percent of all the protein in the particle and said particle is free of pre-S₁ peptide. The particle is useful in the preparation of vaccines against hepatitis B virus.

## Description

### FIELD OF THE INVENTION

The invention relates to Hepatitis B surface antigen ("HBs antigen" or "HBsAG") particles which are composed of polypeptides prepared by recombinant DNA processes, DNA sequences coding for these polypeptides and cell lines for the expression of the same. The present invention relates especially to new particles having increased immunogenicity.

### BACKGROUND OF THE INVENTION

### Expression in Host Cells

Advances in vaccine production techniques have made it possible to synthesize polypeptides corresponding to the HBs antigen in bacteria, yeast and mammalian cells. Transcription of eukaryotic genes in bacteria and yeast, however, adversely affects the efficaciousness of these polypeptides as antigens due to several drawbacks concerning the glycosilation and secretion of the polypeptides and composition of the particle formed therefrom.

For example, in the case of the Hepatitis B virus, the polypeptide antigens produced in vivo are heavily glycosilated (Gerlich, 1984: J. Virol.: 52 (2), 396). In prokaryotes, glycosilation is not an essential process so that polypeptides produced by genetically engineered bacteria are either not glycosilated or are incompletely glycosilated. In either case, polypeptides corresponding to HBsAg, when expressed in bacteria, do not raise antibodies which will see HBsAg sufficiently well for an effective vaccine. Although yeast as a eukaryotic host is capable of more complete glycosilation, polypeptides corresponding to HbsAg expressed in yeast share the same deficiency as in the case of bacterial expression. (Murray et al., 1979: Nature, 282, 575; Valenzuela et al., 1982: Nature, 298, 347; Miyanohara et al., 1983: PNAS, 80, 1).

As a further example, in bacteria the eukaryotic structural gene of the HBsAg is in most cases not efficiently transcribed. Furthermore the structure and function of the eukaryotic HBsAg gene product may be dependent on the additional post-translational processes of the linkage of disulfide bonds which can not be accomplished by the bacterial host.

Still further, the expressed polypeptide is rarely secreted from the bacterial host cells. They must be lysed to harvest the expressed polypeptide. During the purification process bacterial wall components may contaminate the polypeptide and cause serious allergic reactions or lead to anaphylactic shock in patients.

Finally, eukaryotic promoters usually do not work in bacteria and must be substituted by a bacterial promoter which can result in modification of the polypeptide expressed. (Offensperger et al., 1985: PNAS, 82, 7540; Valenzuela et al., 1980: ICN-UCLA Symp, Mol. Cell. Biol., 18 57).

### FORMATION AND SECRETION OF PARTICLES

The natural forms of Hepatitis B virus ("HBV") and HBV protein occur in three distinct morphologies:
- the HBV-virion (Dane particle), which is thought to be the infectious material,
- the filaments, and
- the 20 or 22 nm particles (hereinafter "20 nm particle") which consist only of a protein envelope.

The most interesting form for an efficient vaccine is the 20 nm particle because 1) the coding sequences are entirely known, 2) it is completely uninfectious, and 3) it causes some useful immunogenicity in a human organism.

The three known components of HBV particles differ in their relative amounts of the protein composition. There are three monomers called the major protein with 226 amino acids, the middle protein with 281 amino acids, and the large protein with 389 or 400 amino acids, depending on the subtype ayw and adw, respectively. The large protein is encoded by the complete sequence of the pre-S₁-, pre-S₂- and S-regions, whereas the middle protein is derived from only the pre-S₂- and S-regions, and finally the major protein from only the S-region (Tiollais et al., 1985: Nature, 317, 489; Dubois et al., 1980: PNAS, 77, 4549; McAlzer et al., 1984: Nature, 307, 178).

The infectious virion of HBV (Dane particle) contains 40-80 times more of the high molecular monomers - the pre-S₁ and pre-S₂ peptides - compared to the 20 nm particle. It is now known that these pre-S polypeptides may be associated with some biological and clinical implications. The polyalbumin receptor on the pre-S polypeptides can bind polymerized albumins from humans and chimpanzees which are susceptible to HBV (Thung et al., 1983: Liver, 3, 290; Machida et al., 1984: Gastroenterology, 86, 910). This narrow host range and the known receptor for poly human serum albumin on human hepatocytes explain the hepatotropism of HBV: Dane particles are able to contact hepatocytes via poly human serum albumin taken up by hepatocytes from circulation. Based on this evidence the pre-S peptides should be helpful for an efficient vaccine against HBV because its antibody could be expected to block the significant site on Dane particles that are required for entering hepatocytes (Tiollais et al., 1985: Nature, 317, 489; Millich et al., 1985: Science, 228, 1195).

Literature data would also suggest a better protection against the infectious Dane-particle where the pre-S₁ epitope is present in much higher ratio than on the envelope particles.

The vaccine obtained from natural sources (e.g., donor blood), which causes a limited immunogenic protection, contains (almost) none of the pre-S proteins; this is due to two different reasons. First, the purification process is focused on the noninfectious 20 nm particles. These contain at most 1% pre-S₁ peptide compared to 15-20% in the Dane particle (Gerlich, 1984: J. Vir., 52 (2), 396; Tiollais et al., 1985: Nature, 317, 489; Gerlich, 1982: virology, 123, 436). Second, the 20 nm particles are isolated from sera of anti-HBE positive carriers (Hevac B, HepaVac B) or are digested by proteases during the purification process. This proteolytic digestion has been shown to cut the pre-S-polypeptides leaving only the S monomers. As a result these vaccines contain none or very little pre-S polypeptides.

Therefore there is a demand for a vaccine in the form of HBs antigen particles which possess a high immunogenicity due to the composition of the particle, which undergo glycosilation in the cell and which are secreted continuously from the particle-producing cell.

### REFERENCES AND PATENTS

EP-A-72 318 describes the expression of HBsAg in yeast cells, which have been transformed by a vector comprising a yeast replicon, a yeast promoter and a DNA sequence coding for the S peptide.

Laub et al., J. Virol., Vol. 48, No. 1, pp. 271-280, 1983, disclose the construction of a vector starting from simian virus 40 into which the HBsAg including the 163 codon precursor sequence was incorporated. Laub et al. report that CV-l cells transformed with said vector yield a better expression when the vector contains only the coding sequence for the S protein as compared to the above vector which comprises additionally also the 163 codon precursor sequence.

Also Takeda Chemical Ind., Japanese Patent Application No. J5-8194-897-A describes the expression of the entire pre-S and S peptides. Reference is also made to the expression of the adw subtype.

Feitilson et al., Virology, Vol. 130, pp. 75-90, 1983, have described the partial expression of polypeptides within the pre-S coding sequence, including species with 24000, 28000, 32000, 43000 and 50000 dalton.

Further, DE-OS 34 39 400 describes the expression of an immunogenic polypeptide sequence of Hepatitis B virus.

Said sequence represents a partial sequence of the pre-S₁ polypeptide, comprises 108 or 119 codons and starts with the first starting codon of HBsAg, and terminates 281 codons in front of the stop codon.

EP-A-154 902 discloses a Hepatitis B vaccine which contains a peptide with an amino acid chain of at least six consecutive amino acids within the pre-S chain coding region of the envelope of Hepatitis B virus. This vaccine is free of an amino acid sequence corresponding to the naturally occurring envelope proteins of Hepatitis B virus.

Also Kent et al. have described in Pept. Chem., Vol 22, pp. 16770, 1984, that a chemically synthesized peptide comprising the N-terminal 26 amino acids of the pre-S₂ region can serve as an antigen and may therefore be suitable as a synthetic vaccine.

### OBJECTS OF THE INVENTION

None of the above discussed references consider the possibility that, by altering the composition of the monomers making up the 20 nm particles and approaching thereby the natural composition of the Dane particle, the antigenicity of the particle can be improved.

As discussed mentioned above, the immunogenicity of the peptide monomers of the virus envelope protein is very poor compared to assembled protein particles. The object of this invention is the development of protein particles which contain an amount of the pre-S polypeptide epitopes comparable to the natural composition of the surface structure of the infectious Dane particle.

It is a further object to utilize additional pre-S peptides containing important protective epitopes in the development of a better immune response, a longer protection and lower non-responder rate as compared to all the other products either already marketed or under development.

It is a further object to express HBsAg in mammalian cells. This requires overcoming known difficulties where expression of the desired peptide in a mammalian cell can result in:
- different regulatory mechanisms for the three translational/(transcriptional) products
- promoter-promoter inhibition
- different strength of the start codons
- not all peptides expressed.

### SUMMARY OF THE INVENTION

The term "HBV S peptide" as used herein refers to the peptide encoded by the entire S region of the HBV genome. The term "HBV pre-S₂ peptide" as used herein refers to the peptide encoded by the entire pre-S₂ and S regions of the HBV genome. The term "HBV pre-S₁ peptide" as used herein refers to the polypeptide encoded by the entire pre-S₁, pre-S₂ and S regions of the HBV genome. The term "epitope" as used herein refers to a sequence of at least six consecutive amino acids encoded by the designated genome region (e.g., a "HBV pre-S₂ epitope" refers to a sequence of at least six amino acids encoded by the pre-S₂ region of the HBV genome). As used herein "antigenicity" means the ability to provoke an immune response (e.g., acting as a vaccine or an antigen), the ability to cause the production of antibodies (e.g. acting as an antigen) and/or the ability to interact with a cell surface receptor so as to enhance an immune response or production of antibodies (e.g., reacting with a T-cell surface receptor to enhance immune response).

The term "HBV" means any subtype of the virus, particularly adw, ayw, adr and ayr, described in the literature (P. Valenzuela, Nature Vol. 280, p. 815 (1979), Gerlich, EP-A-85 111 361, Neurath, EP-A-85 102 250). Examples of peptide sequences thereof, from which the epitopes of this invention can be derived, are shown in Figures XVI to XX.

In accordance with the present invention, recombinant DNA molecules are disclosed which comprise a first DNA sequence and a second DNA sequence. The first DNA sequence encodes for expression of an amino acid sequence a portion of which displays the antigenicity of an epitope selected from the group consisting of an HBV pre-S₁ epitope and an HBV pre-S₂ epitope. The second DNA sequence encodes for expression of a peptide which upon secretion will form particles which are at least 10 nm in diameter. These particles are believed to be the smallest particles which will effectively form a good vaccine. Preferably the peptide which upon secretion will form particles which are at least 10 nm in diameter is either HBV S peptide, HBV core antigen, polio surface antigen, Hepatitis A surface antigen, Hepatitis A core antigen, HIV surface antigen and HIV core antigen. A substantial portion or all of the HBV S peptide is especially preferred as the peptide encoded by the second DNA sequence. In the recombinant DNA molecules encoding for the first and second DNA sequences must be (1) in the same reading frame, (2) encode for respective discrete regions of a single peptide, and (3) be operatively linked to an expression control sequence. Finally, these recombinant DNA molecules are free of DNA sequences encoding for the expression of the entire HBV pre-S₁ peptide or HBV pre-S₂ peptide.

Specific recombinant DNA molecules of the present invention are also disclosed wherein the first DNA sequence comprises a nucleotide sequence corresponding to the nucleotide sequence of (1) the HBV pre-S₁ and pre-S₂ regions from which the pre-S₂ start codon ATG has been deleted, (2) the HBV pre-S₁ and pre-S₂ regions and wherein the sequences flanking the pre-S₁ ATG have been changed from the natural sequence, (3) the HBV pre-S₁ and pre-S₂ regions and wherein the sequences flanking the pre-S₂ ATG have been changed from the natural sequence, (4) the HBV pre-S₁ and pre-S₂ regions and wherein the 5' terminus of the pre-S₁ region has been deleted, (5) the HBV pre-S₁ and pre-S₂ regions and wherein the 5' terminus of the pre-S₂ region has been deleted, (6) the HBV pre-S₁ region and wherein the 3' terminus of the pre-S₁ region has been deleted, (7) the HBV pre-S₂ region and wherein the 3' terminus of the pre-S₂ region has been deleted, (8) the HBV pre-S₁ and pre-S₂ regions from which the pre-S₂ ATG has been deleted and the second DNA sequence comprises a sequence corresponding to the nucleotide sequence of the HBV S region from which the S ATG has been deleted, and/or (a) an oligonucleotide described in Table I.

Host cells transfected with the recombinant DNA molecules of the present invention are also disclosed. As used herein, "transfected" or "transfection" refer to the addition of exogenous DNA to a host cell whether by transfection, transformation or other means. Host cells include any unicellular organism capable of transcribing and translating recombinant DNA molecules including without limitation mammalian cells, bacteria and yeast. Host cells of the present invention may also be cotransfected with a second recombinant DNA molecule comprising a DNA sequence encoding for expression of an amino acid sequence corresponding to a substantial portion or all of the amino acid sequence of the HBV S peptide.

Peptides are also disclosed comprising a first discrete region and a second discrete region. The first region displays the antigenicity of an epitope of an HBV pre-S₁ epitope or an HBV pre-S₂ epitope. The second region correspond to a substantial portion of a peptide which upon secretion will form particles which are at least 10 nm in diameter. Preferably the peptide which upon secretion will form particles which are at least 10 nm in diameter is either HBV S peptide, HBV core antigen, polio surface antigen, Hepatitis A surface antigen, Hepatitis A core antigen, HIV surface antigen and HIV core antigen. A substantial portion or all of the HBV S peptide is especially preferred. Preferably, the first region is located closer to the N-terminus of the peptide than the second region.

Immunogenic particles are also disclosed which comprise a plurality of first peptide monomers. Each of said first peptide monomers comprises a first discrete region and a second discrete region which can be the same as the first and second discrete regions of the peptides described above. Immunogenic particles are also disclosed which further comprise a plurality of second peptide monomers arid wherein the first and second peptide monomers are bound together by interactive forces between the monomers. Each of said second peptide monomers comprising an amino acid sequence corresponding to a substantial portion of or all of the amino acid sequence of the HBV S peptide.

Immunogenic particles are also disclosed which contain substantially more than one percent, preferably more than five percent, of the pre-S₁ epitope. As used herein, a particle "contains one percent" of a designated epitope if peptide monomers having the designated epitope constitute one percent of all protein in the particle. Immunogenic particles which contain substantially more than ten percent, preferably more than fifteen percent, of the pre-S₂ epitope are also disclosed.

Pharmaceutical preparations and preparations useful for production of antibodies comprising the above-described immunogenic particles in sufficient concentration to elicit an immune response upon administration of said preparation and a suitable carrier are also disclosed. Suitable carriers are known to those skilled in the art and may include simple buffer solutions.

Other preparations useful for production of antibodies are disclosed comprising the above-described immunogenic particles in sufficient concentration to elicit an immune response upon administration of said preparation and a suitable carrier. Suitable carriers are known to those skilled in the art and may include simple buffer solutions.

A process for producing a transfected host cell is disclosed which comprises providing host cells which have been made competent for uptake of DNA, exposing the host cells to a first preparation of DNA comprising one of the above-described recombinant DNA molecules, allowing under suitable conditions the host cells to take up DNA from the first preparation of DNA, and selecting for host cells which have taken up exogenous DNA. The process may further comprise exposing the host cells to a second preparation of DNA comprising a DNA molecule encoding for a peptide including the amino acid sequence of the HBV S peptide and allowing under suitable conditions the host cells to take up DNA from the second preparation of DNA. The exposure and uptake of the second preparation of DNA can be done before or after exposure to and uptake of the first DNA preparation. Alternatively, the first DNA preparation can also include a DNA molecule encoding for a peptide including the amino acid sequence of the HBV S peptide.

A method for producing a peptide is also disclosed which comprises preparing an above-described recombinant DNA molecule, transfecting a host cell with the recombinant DNA molecule, culturing the host cell under conditions allowing expression and secretion of protein by the host cell, and collecting the peptide produced as a result of expression of DNA sequences within the recombinant DNA molecule. The peptide produced by such method can contain less than the entire amino acid encoded by the coding region of the recombinant DNA molecule. This may result from transcription and/or translation of only a portion of the coding region of the recombinant molecule or by deletions made in the peptide after translation.

A method of producing immunogenic particles is disclosed comprising preparing an above-described recombinant DNA molecule, transfecting a host cell with the recombinant DNA molecule, culturing the host cell under conditions allowing expression and secretion of protein by the host cell, and allowing under suitable conditions the aggregation of peptide monomers produced as a result of expression of exogenous DNA sequences within the host cell. A method of producing immunogenic particles is also disclosed which further comprises transfecting (cotransfection) the host cell with a DNA molecule encoding for a peptide including the amino acid sequence of the HBV S peptide. The cotransfection can occur before, after or simultaneous with the transfection of the above-described recombinant DNA molecule. Presence of peptides encoded by the cotransfected DNA molecule are necessary to obtain more than trace amounts of particles secreted from the host cell.

Methods of manufacturing a pharmaceutical preparation and a preparation useful for production of antibodies are disclosed comprising preparing an above-described recombinant DNA molecule, transfecting a host cell with the recombinant DNA molecule, culturing the host cell under conditions allowing expression and secretion of protein by the host cell, allowing under suitable conditions the aggregation of peptides produced as a result of expression of DNA sequences within the host cell to form immunogenic particles, and combining the immunogenic particles with a suitable carrier such that the immunogenic particles are present in sufficient concentration to cause production of antibodies upon administration of a preparation to an individual. Host cells used in these methods can also be cotransfected as previously described.

### BRIEF DESCRIPTION OF THE FIGURES

Figures I to X show gene constructs according to the present invention.

Figures XI and XII show the results obtained by caesium chloride sedimentation of immunogenic particles according to example 10.

Figure XIII shows a prior art construct shown in Table XI.

Figure XIV shows the characterisation of particles derived from a construct according to a further embodiment of the present invention.

Figure XV shows the oligo sequence of Figure X-2.

Figure XVI to XX show examples of peptide sequences from which the epitopes of this invention can be derived.

### DESCRIPTION OF THE PREFERRED EMBODIMENTS

Preferred DNA constructs of the present invention are characterized by the presence of a selection marker selected from the group consisting of dhfr (dihydrofolate reductase), MT-neo (a neomycin resistance sequence coupled to a methallothionein and MT-ecogpt (a resistance sequence coupled to a methallothionein promoter). The expression rate may be further enhanced by adding to the constructs a dhfr gene as an amplification gene.

HBV nucleotide sequences used in certain constructs of the present invention can be formed or isolated by any means including isolation and ligation of restriction fragments and synthetic oligonucleotides. Constructs specifically described herein were formed by the ligation of synthetic oligonucleotides to a 5' XbaI-BglII 3' fragment from the S region of the HBV genome shown in Figure IX (hereinafter the "XbaI-BglII fragment") which is derived from a BglII-BglII HBV fragment including the entire pro-S₁-pre-S₂-S regions (the "Bg1II-Bg1II Fragment"). The pre-S₁-pre-S₂-S region of the HBV genome is shown in Figure IX. Oligonucleotides used in making such constructs are summarized in Table I.

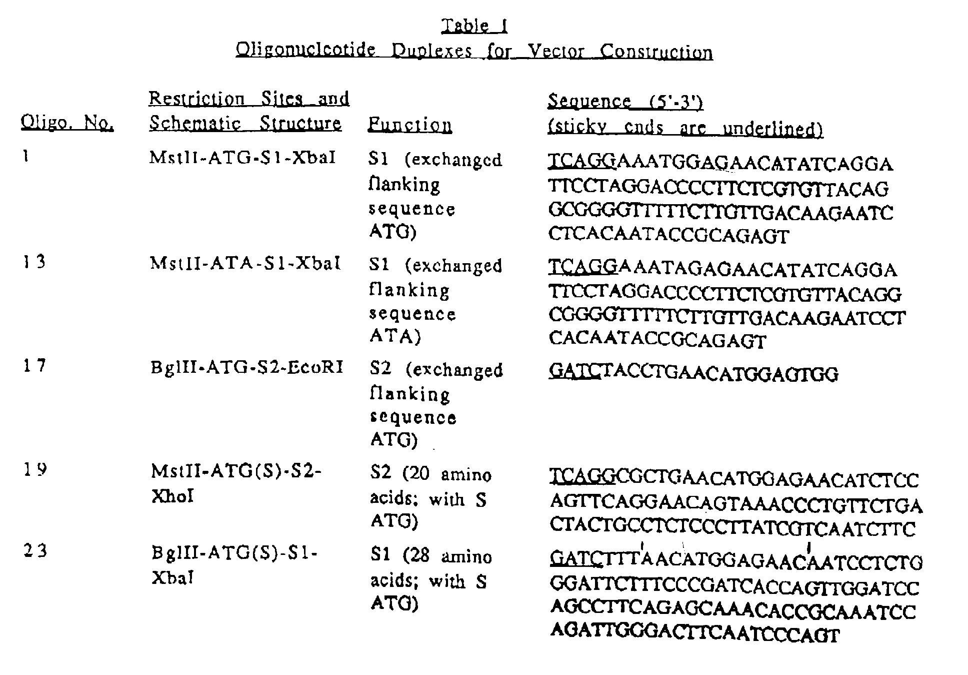

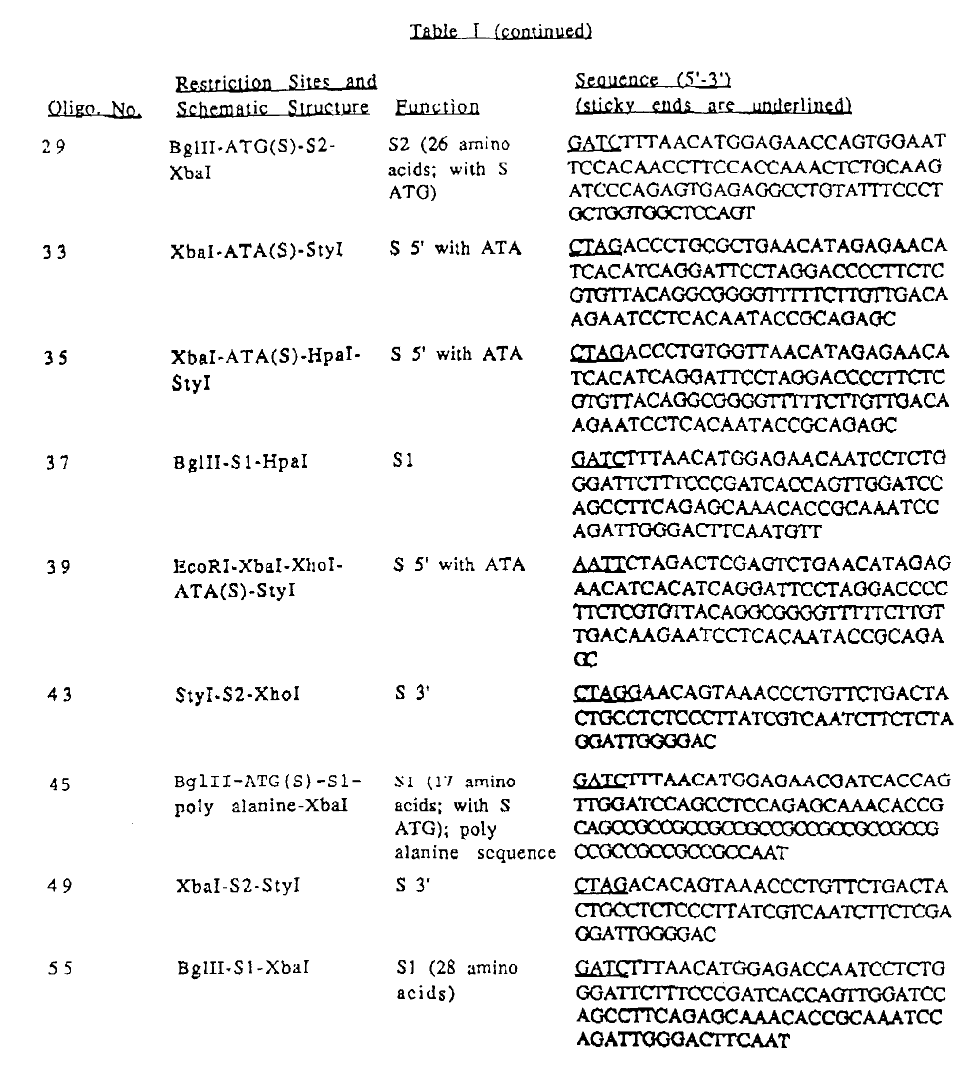

The oligonucleotides in Table I were combined with the XbaI-BglII fragment to produce constructs with desired features. In certain constructs adapter oligonucleotide sequences (Table II) were used to create proper matching sticky ends on the oligonucleotides and other construct components.

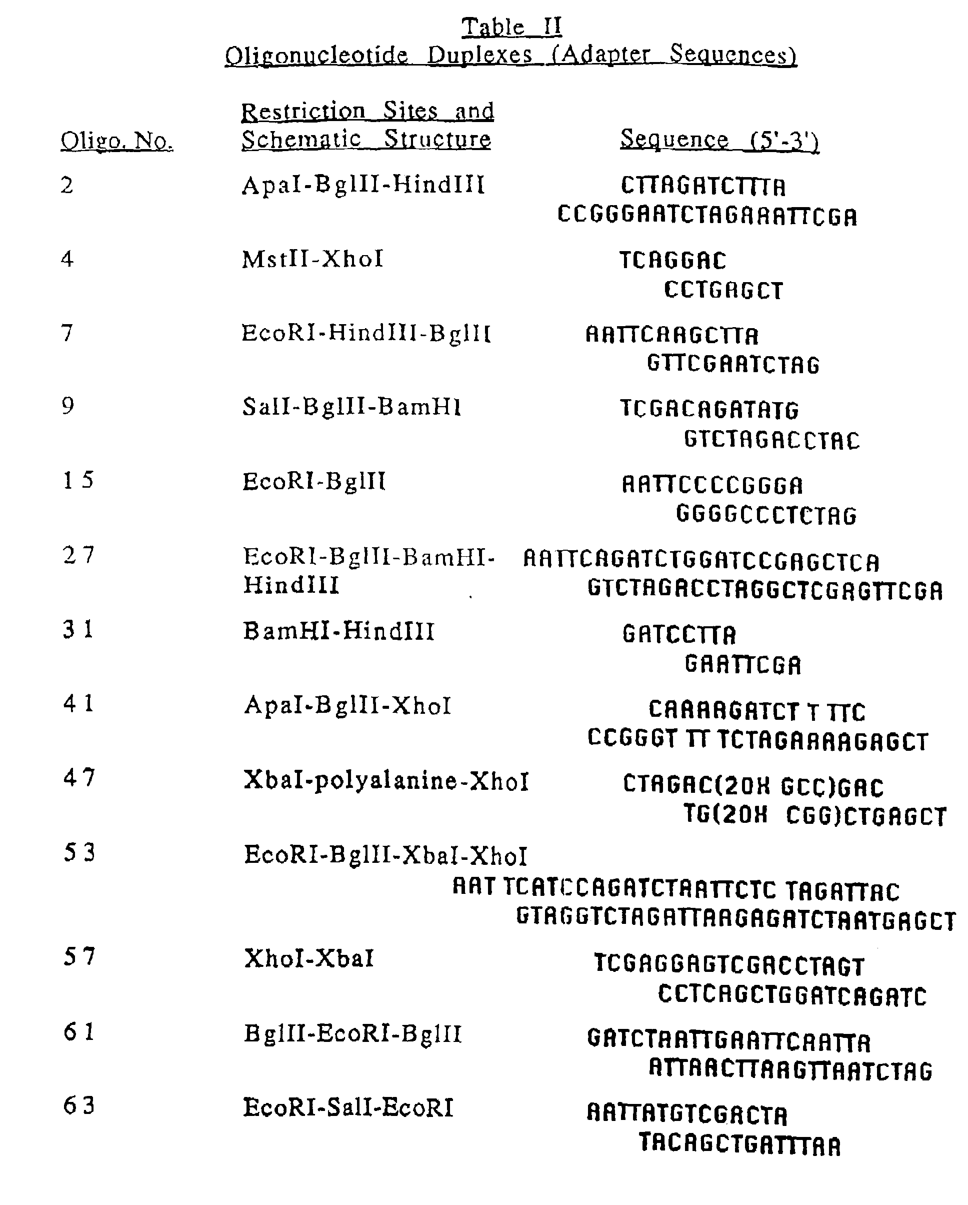

Other adapter sequences may be used to combine desired oligonucleotides from Table I with the XbaI-BglII fragment, other restriction fragments, oligonucleotides and other construct components. The necessary sequences of such other adapter sequences will be readily apparent to those skilled in the art from consideration of tables of restriction sites [e.g., that found at pages 121-128 of Methods in Enzymology, volume 152, "Guide to Molecular Cloning Techniques," ed. Berger and Kimmel (Academic Press 1987) which is incorporated herein in its entirety by reference] and the sequences of the various nucleotides to be combined. Adapter sequences can also be used to introduce additional restriction sites into constructs of the present invention. It should be noted that adapter sequences must be selected or designed so that the proper reading frame is maintained throughout the HBV sequence.

Preferred gene constructs which were used to transfect host cells were prepared by recombinant DNA techniques in accordance with the present invention. Preferred embodiments of constructs with an enhanced expression rate are shown in Figures I-VIII and are schematically represented by the following:
pU2-structural gene
pU2-structural gene-dhfr
pU2-structural gene-dhfr-MT-neo
pU2-structural gene-dhfr-MT-egpt
pMT-structural gene-dhfr
pMT-structural gene-dhfr-MT-neo
pMT-structural gene-dhfr-MT-egpt
pH2K-structural gene-dhfr
pH2K-structural gene-MT-neo
pH2K-structural gene-MT-egpt
pH2K-structural gene-dhfr-MT-neo
pH2K-structural gene-dhfr-MT-egpt

Each of the constructs shown in Figures I-VIII contain, in addition to a HBV sequence, a neomycin selection marker with the MT promoter, an ampicillin selection marker, a dhfr selection/amplification gene and a promoter for the HBV sequence. The promoter for the HBV sequence is preferably the U2 promoter, the MT promoter or the H2K promoter. Isolation of fragments containing the various promoters, the selection markers and amplification gene is described below. The HBV sequences in the constructs of Figures I-VIII are schematically represented by a rectangular bar in each figure which indicates the oligonucleotides and/or adapter sequences from Tables I and II which were combined with the XbaI-BglII fragment. Shaded areas within the bar indicate generally regions of the entire pre-S₁-pre-S₂-S region which are not found in the specific construct. Oligonucleotides from Table I which can be used to construct each type of HBV sequence are indicated in the figures.

Figure X depicts two additional constructs for expression of peptides including sequence from the pre-S2 region under the control of the MT promoter.

Constructs have also been made which include the entire BglII-BglII fragment from the HBV genome under the control of the US promoter. These constructs have produced peptides which include a deletion in the S region as indicated by Western blot analysis.

The above-cited promoters are specially preferable when their use is coupled with a modulation method using the dhfr gene and methotrexate to enhance the expression. This is achieved when in addition to the selection marker the dhfr minigene is also introduced into the plasmid sequence. It is essential that the dhfr gene is located on the same plasmid together with the structural gene to be expressed. An enhancement of the expression rate of the structural gene can then be obtained by adding methotrexate in the micromolar concentration range. Thereby a manyfold enhancement of the expression rate is achieved.

Suitable cells are e.g. VERO cells (monkey kidney cell line), 3T3-cells (murine fibroblast line), C127-cells (murine fibroblast line), L-cells and CHO - cells (Chinese hamster cells, which are either positive or negative in dehydrofolate reductase).

As a stop signal it is preferred to use a stop signal from a eukaryotic cell. Preferably the stop signal of the caseine DNA-sequence is used. As used throughout the following examples, "HBV protein" refers generically to any protein produced in accordance with the present invention which corresponds to HBsAg sequences.

### EXAMPLE 1

### Particle Purification Procedures

### 1. Fractionated precipitation with polyethylene glycol (PEG)

The supernatant of HBV protein producing cultures was collected and split into portions of 2,400 ml. To each portion 144 g of PEG 6000 (Serva) were added and dissolved by stirring at room temperature for 20 minutes and was stirred for another 6 hours at 4°C. The precipitate was separated by centrifugation in 500 ml bottles in a GS 3 rotor at 9,000 rpm (15,000 x g) for 30 minutes at 10 C. The supernatant was collected and 144 g of PEG 6000 were added and dissolved as described above. The solution was stirred at 4 C for 3 hours. The precipitate from this solution was harvested as described above except that centrifugation was continued for 60 minutes.

### 2. Gel Chromatography

The material obtained after PEG precipitation was redissolved in 20 ml PBS and submitted to gel chromatography on A-5m (BioRad). Column dimensions were 25 x 1000 mm and 480 ml bed volume. In a typical fractionation run 1,000 ug of PEG precipitated HBV protein in 10 to 15 ml was loaded and eluted with PBS at a speed of 6 drops/min (18 ml/h) 3 ml fractions were collected. HBV protein eluted with the first peak. Collected fractions were submitted to a CsCl gradient.

### 3. Sedimentation in CsCl Gradient

About 30 fractions covering the first peak in column chromatography on A-5m and containing prepurified HBV protein were collected to approximately 100 ml. This solution was adjusted to a density of 1.30 g/cc with CsCl and subsequently transferred to a nitrocellulose tube fitting into a SW 27/28 rotor (Beckman). A gradient was set by underlaying 4 ml of a CsCl solution of 1.35 g/cc and by overlaying 4 ml of 1.25 g/cc followed by 4 ml of 1.20 g/cc density. This gradient had been run at 28,000 rpm for 50 hours at 10 C. Thereafter the gradient was fractionated and purified HBV protein floating in the 1.20 g/cc density layer was collected. The solution was desalted by three cycles of dialysis in bags against water.

### Example 2

### Quantitative Determination of HBV protein

### 1, with Radioimmunoassay

In the AUSRIA II-125 "sandwich" radioimmunoassay (commercially available from Abbot), beads coated with guinea pig antibody to Hepatitis B Surface Antigen (Anti-HBs) were incubated with serum or plasma or purified protein and appropriate controls. Any HBsAg present was bound to the solid phase antibody. After aspiration of the unbound material and washing of the bead, human 125T-Anti-HBs was allowed to react with the antibody-antigen complex on the bead. The beads were then washed to remove unbound ¹²⁵I-Anti-HBs.
)-Anti-HBs HBsAg
)-Anti-HBs . HBsAg 125I-Anti-HBs
)-Anti-HBs . HBsAg . 125-Anti-HBs

The radioactivity remaining on the beads was counted in a gamma scintillation counter.

### 2. with ELISA

In the Enzygnost HBsAg micro "sandwich" assay (commercially available from Behring), wells were coated with anti-HBs. Serum plasma or purified protein and appropriate controls were added to the wells and incubated. After washing, peroxidase-labelled antibodies to HBsAg were reacted with the remaining antigenic determinants. The unbound enzyme-linked antibodies; are removed by washing and the enzyme activity on the solid phase is determined. The enzymatically catalyzed reaction of hydrogen peroxide and chromogen was stopped by adding diluted sulfuric acid. The colour intensity was proportional to the HBsAg concentration of the sample and was obtained by photometric comparison of the colour intensity of the unknown samples with the colour intensities of the accompanying negative and positive control sera.

### Example 3

Preparation of a construct of the present invention containing the methallothionein promoter.

### 1) Isolation of the MI promoter

The plasmid pBPV-342-12 (commercially available from ATCC) was digested with the endonucleases Bg1II and BamHI. Three DNA molecules were generated. The fragment of interest contains the methallothionein promoter and a pBR322 sequence comprising 4.5 kb and is easily detectable from the other fragments (2.0 kb and 7.6 kb).

The reaction was performed in a total volume of 200 ul of reaction buffer at a final concentration of 0.5 ug/ul DNA including 100 units of each restriction enzyme. The completion of the digestion was checked after incubation at 37°C for three hours by agarose gel electrophoresis at a 0.8% agarose gel. The reaction was stopped by adding 4 ul 0.5 M EDTA.

The 4.5 kb fragment was separated from the other fragments by preparative 1.2% agarose gel electrophoresis. The DNA was eluted from the agarose gel on DE-81 Whatman filter paper from which the DNA was removed in a high salt buffer. The DNA was purified by a phenol/chloroform extraction and two ethanol precipitations.

### 2) Ligation of the 2.3 kb MBV Bg1II-Bg1II fragment

A 2.3 kb BglII-BglII fragment containing the HBV pre-S₁,pre-S₂ and S coding regions was isolated from HBV-containing DNA. The 2-3kb fragment was ligated together with the 4.5 kb fragment (obtained as described in Cl) containing the methallothionein promoter.

2 ul of the 2.3 kb fragment were mixed with 3 ul of the 4.5 kb fragment and ligated together in a total volume of 10 ul ligation buffer, containing 2 units T₄-DNA ligase and 2mM ATP at 14°C overnight.

The ligation mixture was added to 150 ul competent bacterial cell suspension for DNA up-take. After the DNA up-date the bacterial cells were spread on LB agar plate containing 50 ug/ml ampicillin at volumes of 50 to 300 ul cell suspension per plate. The agar plates were incubated at 37°C overnight. Single isolated bacterial colonies were screened for the presence of a plasmid containing the desired fragments.

### 3) Screening for desired plasmid containing bacterial colonies.

Single colonies were picked with a toothpick and transferred to a LB-ampicillin media containing tube (5 ml). The tubes were incubated overnight at 37°C by shaking rapidly. A mini-plasmid preparation of each grown bacterial suspension was made. The different resulting DNAs were proved by digestion with the restriction endonuclease EcoRI. Two molecules were expected, a 2.2 kb fragment and S 4.6 kb fragment. The digestion was analysed by agarose gel electrophoresis. Plasmid DNA was isolated from the bacterial cells.

### 4) Conversion of a part of the HBV-gene sequence.

The plasmid resulting from (3) above was digested with the endonucleases BglII and XbaI. Two molecules were expected, one 550 bp fragment and one 6.250 kb fragment which was isolated after agarose gel electrophoresis.

The 6.250 kb fragment was ligated together with oligomecleotide No.55 from Table I. The ligation mixture was added to 150 ul competent bacterial cell suspension for DNA up-take. Single isolated bacterial colonies were screened for the presence of the desired plasmid. The new plasmid was proved by a digestion with the endonucleases EcoRI and BglII. Two molecules were expected, one 1.9 kb and one 4.450 kb.

### 5) Insertion of a neomycin selection marker.

The plasmid resulting from (4) above was linearized by digestion with the restriction enzyme EcoRI. The reaction was performed in a total volume of 50 ul and a final concentration of 1 ug/ul plasmid DNA. 50 units of ECORI were added and the digestion was proved after incubation at 37°C for three hours by agarose gel electrophoresis. The reaction was stopped by adding 1 ul of 0.5 M EDTA and the DNA was precipitated with a final concentration of 0.3 M sodium acetate and 3-4 volumes of ethanol at -80°C for 30 minutes. The precipitated DNA was dissolved in 50 ul distilled water.

2 ul of the linearized plasmid were mixed with 3 ul of the DNA fragment containing the methallothionein promoter and the neomycin selection gene [isolated from the plasmid pMT-neo-E (available from ATCC ) by digestion with the endonuclease EcoRI as a 4kb fragment], and ligated together. Single bacterial colonies were screened for the presence of the desired plasmid.

### 6) Additional of the dhfr Amplification Gene dhfr

The plasmid pdhfr3.2 (available from ATCC) was digested with the restriction endonuclease HindIII. Two molecules were generated, one of 3,000 bp containing the dhfr gene sequence and one of 3,400 bp. The 3,000 bp fragment was isolated and ligated into the plasmid resulting from (5) above which was previously opened by digestion with HindIII. The resulting plasmid is represented by Fig. I-2.

### Example 4

### 1) Isolation of a fragment containing the U2 promoter sequence.

The plasmid pUC-8-42 (available from Exogene ) was digested with the restriction endonucleases EcoRI and Apal. Two DNA molecules were generated. The fragment of interest contains the U2-promoter comprising 340 bp and is easily detectable from the other fragment (3160 bp). The digestion was performed in a total volume of 200 ul of reaction buffer at a final concentration of 0.5 ug/ul DNA including 100 Units of each restriction enzyme. The completion of the digest was checked after incubation at 37°C for three hours by agarose gel electrophoresis in a 0.7% agarose gel. The reaction was stopped by adding 4 ul 0.5 M EDTA. The 340 bp fragment was separated from the plasmid DNA by preparative 1.2% agarose gel electrophoresis. The DNA was eluted from the agarose gel on DE-81 Whatman filter paper from which the DNA was removed in a high salt buffer. The DNA was purified by a phenol/chloroform extraction and two ethanol precipitations.

### 2) Insertion of the fragment containing the promoter sequence into a polylinker plasmid.

The plasmid pSP165 (commercially available from Promega Biotec) containing a polylinker sequence (containing the following restriction sites: EcoRI, SacI, SmaI, AvaI, BamHI, BglII, SalI, PstI, HindIII) was linearized with the restriction enzyme EcoRI. The reaction was performed in a total volume of 50 ul and a final concentration of lug/ul plasmid DNA. 50 Units of EcoRI were added an the digestion was proved after incubation at 37°C for three hours by agarose gel electrophores. The reaction was stopped by adding 1 ul of 0.5 M EDTA and the DNA was precipitated with a final concentration of 0.3 M sidium acetate and 3-4 volumes of ethanol at -80°C for 30 minutes. The precipitated DNA was dissolved in 50 ul distilled water.

2 ul of plasmid DNA were mixed with 10 ul of the fragment DNA containing the V2 promoter sequence, and ligated together in a total volume of 25 ul of ligation buffer containing 2 units T4-DNA ligase and 2 mM ATP at 14°C overnight. Thereafter the DNA was purified by phenol/ chloroform extractions followed by two ethanol precipitations and dissolved in 10 ul distilled water. The resulting sticky ends of EcoRI and ApaI had to be converted into blunt ends and ligated. The blunt ends were converted by a removing reaction with the Mung bean nuclease as follows: to 25 ul DNA (1 ug/ul concentration) reaction buffer, 20 units of enzyme and a final concentration of 1% glycerol to the reaction volume of 35 ul were added. After an incubation for 30 minutes at 30 C the DNA was purified by phenol/chloroform extractions followed by two ethanol precipitations. The DNA was dissolved again in 5 ul distilled water. The resulting blunt ends were ligated together in 15 ul reaction volume containing 10 x more T4 ligase then used above and 2 mM ATP at 14°C overnight.

The ligation mixture was added to 150 ul competent bacterial cell suspension for DNA up-take. After the DNA up-take the bacterial cells were spread on LB agar plates containing 50 ug/ml ampicillin at volumes of 50 to 300 ul cell suspension per plate. The agar plates were incubated at 37°C overnight. Single isolated bacterial colonies were screened for the presence of a plasmid containing the desired U2-promoter fragment.

### 3. Screening for desired plasmid containing bacterial colonies

Single colonies were picked with a toothpick and transferred to a LB-ampicillin containing tube (5 ml). The tubes were incubated overnight at 37°C by shaking rapidly. A mini plasmid preparation of each grown bacterial suspension was made. The different resulting plasmid was proved by digestion with both restriction endonucleases EcoRI and HindIII. Two molecules were found, a 400 bp fragment containing the U2 promoter sequence and the plasmid of 2,700 bp. The digestion was analysed by agarose gel electrophoresis. The resulting plasmid was isolated from the bacterial cells.

### 4) Insertion of the neomycine selection marker.

The plasmid pBPV-342-12 (commercially available from ATCC) was digested with the endonucleases EcoRI and BamHI. Two molecules were isolated, one containing the MT promoter together with the neomycin selection gene of 4,000 bp and the plasmid of 10,000 bp.

The plasmid resulting from (3) above was linearized with EcoRI and ligated together with the 4,000 bp fragment containing the MT-promoter together with the neomycin selection gene. The resulting sticky ends were also converted into blunt ends and ligated together as described above.

After bacterial transformation, colony selection and mini plasmid preparation, the resulting plasmids were analysed by a digestion with the restriction enzymes ECORI and HindIII. Two DNA molecules were isolated, a 400 bp fragment and a 6,700 bp fragment.

### 5) Ligation of the BglII-BglII fragment

The plasmid resulting from (4) above was linearized with Bg1II. The 2.3 kb-BgIII-BgIII fragment was ligated together with the linearized plasmid. Bacterial colonies were analysed to find the resulting plasmid. The plasmid-DNA was digested with EcoRI and two resulting fragments were obtained, a 700 bp fragment (containing the promoter and a part of the HBV-sequence) and a 8,700 bp fragment (containing the rest of the HBV-sequence, MT-neo and plasmid).

### 6) Alterations within the HBV-sequence

The plasmid resulting from (5) above was digested with the endonucleases BglII and MstII. Two molecules were generated, one of 300 bp containing part of the pre-S sequence and the other (9,100 bp) which was eluted as described above. This 9,100 bp fragment was ligated to another BglII/MstII 216 bp fragment (sequence 
= AGATCTACAGCATGGGCAGAATCTTTCCACCAGCAATCCTCTGGGATTCTTTCCCGACCA
BglII S1
CCAGTTGGATCCAGCCTTCAGAGCAAACACCGCAAATCCAGATTGGGACTTCAATCCCAA
CAAGGACACCTGGCCAGACGCCAACAAGGTAGGAGCTGGAGCATTCGGCCTGGGTTTCAC
CCCACCGCACGGAGGCCTTTTGGGGTGGAGCCCTCAGG )
MstII
 coding for an altered pre-S₁ gene sequence.

The desired plasmid was digested with EcoRI and two resulting fragments were isolated, a 616 bp fragment and a 8,700 bp fragment.

### Example 5

### Isolation of the H2K Promoter

The H2K promoter was isolated as an EcoRI/BglII fragment (2kb) from psp65H2 (available from Exogene).

### Isolation of the egpt selection marker

The fragment containing the methallothionein promoter and the egpt-selection gene was isolated by digestion of the plasmid pMSG (available from Pharmacia) with the restriction enzyme EcoRI as a 3.6 kb fragment.

All other plasmid constructions were made in similar ways by combining fragments containing the necessary components and employing desired oligonucleotides and adapter sequences (where necessary).

### Example 6

### Transfection of Mammalian Cells with Constructs of the Present Invention.

In order to achieve secretion of substantial amounts of the HBV peptides encoded by constructs of the present invention, mammalian cells must be transfected with both the construct of the present invention and a construct which will express entire S protein. The cotransfection was performed in two steps (i.e., a separate transfection for each construct) or in a single step (i.e., one transfection using preparation of both constructs). Cotransfection was confirmed either by use of different selection markers on the two constructs or by detection of secretion of expression products of both constructs by immunoassay.

Alternatively, a sequence encoding the HBV peptide sequence of the present invention and a separate sequence encoding the entire S protein could be combined in a single construct.

### Example 7

### General Procedures

General procedures useful in practicing the present invention may be found in (1) Methods of Enzymology, volume 152, "Guide to Molecular Cloning Techniques," ed. Berger and Kimmel (Academic Press 1987), and (2) Maniatis et al., "Molecular Cloning: A Laboratory Manual," (Cold Spring Harber Laboratory 1982), both of which are incorporated herein in their antirety by reference. Specific techniques employed are described below.

### 1) Digestion with Endonucleases and Isolation of Fragments

The restriction endonucleases used were:
BglII, BamHI, HindIII, EcoRI, XbaI, MstII, XhoI, PflMI, commercially available from Gibco/BRL with their respective restriction buffers (10x).

Unless otherwire indicated, restriction digests were performed and fragments were isolated as follows.
Reactions typically contained 1-5 ug DNA.
- distilled water was added to the DNA in an eppendorf tube to a final volume of 8 ul
- 1 ul of the appropriate 10x digestion buffer was added
- 1 ul (containing 5-10 U) restriction enzyme was added and mixed carefully
- the reaction tube was incubated for 1 hour at 37°C
- digestion was stopped by adding 0.5 M EDTA (pH 8.0) to a final concentration of 10 mM
- if the DNA was analysed directly on a gel, 1 ul of gel-loading dye III (Maniatis) was added, mixed and the sample was loaded into the slots of a 0.8% agarose gel.

The agarose gel normally contains 0.8% agarose 1 x running buffer (TBE, Maniatis). Where a fragment (about 100-1000bp) was isolated from an agarose gel the agarose was increased to 1.2 to 1.4%.

### 2) Competent Bacterial Cells

From a dense overnight culture, 1 ml of the bacterial cell suspension was added to 100 ml fresh growth medium (L-broth). The cells were grown at 37°C to a density of OD₆₀₀ = 0.7 which was reached within 2 hours with vigorous shaking in a 500 ml Erlenmeyer flask. Growth was stopped by chilling the culture on ice for 10 minutes. From this culture, 3 ml were taken for harvesting the exponential bacterial cells at 3,000 rpm for 5 minutes. The cells were resuspended in 1.5 ml of 50 mM CaCl₂ in 10 mM Tris, pH 8.0, and incubated on ice for another 15 minutes. The cells were harvested once more by centrifugation at 3,000 rpm for 5 minutes and resuspended in 200 ul of 50 mM CaCl₂ in 10 mM Tris, pH 8.0, and used directly.

### 3) Transformation of Competent Bacterial Cells

The DNA to be transformed was suspended in 10 mM Tris, pH 7.5, 1 mM EDT 70 ul and added to the 200 ul bacterial cell suspension for DNA take-up. The mixture was incubated on ice for 30 minutes and then 1 ml L-broth was added. The mixture was incubated at 42°C for 2 minutes and at 37°C for 40 minutes.
After the incubation, the cells were spread on agar plates containing 50 ug ampicillin/ml agar at volumes of 50-300 ul cell suspension per plate. The agar plates were incubated at 37°C overnight. After this incubation period, single isolated bacterial colonies were formed.

### 4) Plasmid DNA Isolation

1 liter of plasmid-bearing cells was grown to 0.5 OD₆₀₀ in L-broth and amplified for 20 hours with 200 ug/ml chloramphenicol. The culture was then centrifuged at 4,000 rpm for 20 minutes in JA-10 rotor, 4°C. The pellet was resuspended in 18 ml cold 25% sucrose, 50 mM Tris, pH 8.0, transferred to a 250 ml Erlenmeyer flask and kept on ice. 6 ml 5mg/ml lysozyme in 250 mM Tris, pH 8.0 was added and the mixture was left to stand 10-15 minutes. 6 ml 250 mM EDTA, pH 8.0, was added, mixed gently and incubated for 15 minutes on ice. 30 ml detergent (0.01% Triton X-100; 60 mM EDTA, pH 8.0; 50 mM Tris, pH 8.0) was added and the mixture was incubated for 30 minutes on ice. After incubation, the mixture was centrifuged at 25,000 rpm 90 minutes in SW28 rotor, 4°C.

Pronase was added to supernatant fluid to 250 ug/ml and incubated 30 minutes, 37°C. The solution was extracted with phenol once with 1/2 volume phenol equilibrated with 10 mM Tris, pH 8.0, 1 mM EDTA. The aqueous layer was removed. Sodium acetate was then added to a final concentration of 300 mM, followed by the addition of 3 volumes cold 100% ethanol and thorough mixing. The mixture was stored at -20°C overnight.

The mixture was thawed and centrifuged. The pellet was resuspended in 6 ml 10 mM Tris, 10 mM EDTA, pH 8.0. 9.4 g CsCl and 0.65 ml of 6 mg/ml ethidium bromide were added and the volume was brought up to 10 ml with sterile double-distilled water. The 10 ml alignots were put into Beckman heat-sealable gradient tubes and centrifuged, 50,000 rpm, 48 hours in Ti70.1 Beckman rotor.

Plasmid bands were visualized with UV and removed with syringe and 18 gauge needle by piercing the side of the tube. Ethidium bromide was removed from the plasmid fractions by 3 successive extractions with equal volumes of isobutanol. Fractions were then (1) dialyzed against one 2-liter lot of 10 mM Tris, pH 7.4, 1 mM EDTA, pH 7.5, 5 mM NaCl for 2 hours or more at 4°C; and (2) phenol extracted once with 1/3 volume phenol equilibrated as above. Sodium acetate was then added to a final concentration of 300 mM, followed by addition of two volumes of 100% ethanol. Precipitate formed at -20°C overnight, or at -70°C for 30 minutes.

### 5) Mini-Plasmid Preparation

1 ml of an overnight bacteria culture was put into an eppendorf tube and centrifugated for 20 minutes. The supernatant was removed. 100 ul of 50 mM glucose, 25 mM Tris (pH 8.0), 10 mM EDTA (pH 8.0) was added to the pellet, mixed by vortex and incubated for 5 minutes at room temperature. 200 ul of 0.2 N NaOH, 1% SDS was added, mixed by vortex and incubated for 5 minutes on ice. 150 ul 3 M Sodium acetate (pH 4.8) was added, mixed by vortex and incubated for 5 minutes on ice. After centrifugation for 5 minutes at 13,000 rpm the supernatant was decanted into a fresh eppendorf tube. 3 volumes of 100% ethanol were supplemented, mixed well and incubated for 30 minutes at -80°C, then centrifuged for 10 minutes at 13,000 rpm. The ethanol was removed, the pellet washed with 70% ethanol, lyophilized and dissolved in 20 ul distilled water. 5 ul of this plasmid DNA solution were used directly for restriction analysis.

### 6) Nick Translation

Nick translation was performed according to Rigby et al., J. Mol. Biol., Vol. 113, pp. 237-251, 1977, which is incorporated herein by reference. The reaction mixture for ³²P-labeling of DNA contained 0.5 ug of a HBV fragment, in a total volume of 30 ul with 50 mM Tris, pH 7.8, 5 mM MgCl₂, 10 mM mercaptoethanol, 0.1 mM dATP, 0.1 mM dGTP, 0.1 mM dTTP, 50 uCi ³²P-dCTP, 10 units DNA polymerase I, 3 ul of a 2 x 10⁻⁵ fold dilution of 1 mg/ml DNase I and is incubated for 90 minutes at 15°C, yielding 3 x 10⁶ to 12 x 10⁶ total cpm, i.e. 1 x 10⁷ to 5 x 10⁷ cpm/ug DNA.

### 7) Southern Blot Analysis

To characterize the organization within the host cell genome of the vectors of this invention, chromosomal DNA from cell lines producing particles of this invention were isolated and digested with the appropriate restriction enzyme(s) and analysed by the method of Southern (J. Mol. Biol., Vol. 98, pp. 503-517, 1975), which is incorporated herein by reference, using a ³²P-labeled DNA probe.
Following digestion of the chromosomal DNA (20 ug) with the restriction enzyme BglII, the resulting fragments were separated by 0.7% agarose gel electrophoresis. Thereafter, the DNA was denatured by exposing to 366 nm UV light for 10 minutes and by incubation in a solution of 0.5 M NaOH and 1 M NaCl for 45 minutes. The gels were neutralized by incubation in 0.5 M Tris, 1.5 M NaCl, pH 7.5 for 60 minutes. The DNA was transferred to a nitrocellulose filter by soaking in 3 M NaCl, 0.3 M Sodiumcitrate (20 x SSC) for 20 hours through the gel by covering the top of the nitrocellulose filter with a staple of dry paper towels. The nitrocellulose filter was kept for 2 hours in a vacuum oven at 80 C. A radioactive DNA probe from the BglII fragment of the pHBV (2.3 kb) was prepared by nick translation.
For hybridization with the DNA probe, the nitrocellulose filter was sealed in a plastic bag containing 10 ml of prehybridization mixture: 50% formamide, 5 x SSC, 50 mM Sodiumphosphate, pH 7.0, 5 x Denhardt's solution, 250 ug/ml denatured salmon sperm DNA. The filter was incubated in this mixture for 4 hours at 45°C, after which the pre-hybridization mixture was replaced by the hybridization mixture: 50% formamide, 5 x SSC, 20 mM Sodiumphosphate, pH 7.0, 1 x Denhardt's solution, 100 ug/ml denatured salmon sperm DNA, 5 x 10⁵ cmp/ml ³²P-probe. The filter, after incubating in the hybridization mix for 18 hours at 45°C, was washed three times, 5 minutes each, in 0.1 x SSC, 0.1% SDS at 50°C. The filter was dried at 60°C for 10 minutes and exposed to two X-ray films (XAR-5, KODAK) between two intensifying screens and kept at -80°C. The first X-ray film is developed after 3 days' exposure; the second film after 7 days' exposure.

### 8) Preparation of Mammalian Cells and DNA Precipitate for Transfection

The recipient cells (C127 or CHO-cells available from ATCC) were seeded in normal growth medium (DMEM+10% Fetal Calf Serum, Glycose and Glutamin) into petri-dishes (1-2 x 10⁶ cells per dish, φ 10 cm) at day 1. The next day the medium was removed (4 hours before the DNA precipitate was added onto the cells), and the cells were washed twice with 1 x PBS. Then 8 ml DMEM without FCS were added. 4 hours later the DNA precipitate (prepared as described below) was added to the cells. Again after 4 hours the medium was removed, 3 ml of Glycerol-Mix (50 ml 2 x TBS buffer, 30 ml glycerol, 120 ml distilled water) were added. The Glycerol-Mix was immediately removed after an incubation at 37°C for 3 minutes and the cells were washed with 1 x PBS. The cells were cultivated overnight with 8 ml of DMEM with 10% FCS.

After 48 hours, the cells were recovered from the dish by treating with Trypsin-EDTA-Solution (0.025% Trypsin + 1 mM EDTA). Afterwards, to remove the Trypsin-EDTA the cells were washed with 1 x PBS, suspended in DMEM with 10% FCS and distributed into 24 costar-well-plates (cells from one dish into four 24-well-plates). When the cells had grown well, selection medium was added (concentration 0.5 - 1mg/ml of neomycin,or xanthine: 250 µg/ml, hypoxanthine: 15 µg/ml (or adenine: 25 µg/ml), thymidine: 10 µg/ml, aminopterine 2 µg/ml mycophenolic acid: 25 µg/ml for eco-gpt, for example). The medium was changed every week. The first growing cell colonies were seen after 2 weeks.

To 10 ug of plasmid DNA and 20 ug of carrier-DNA (salmon-sperm DNA, calf-thymus DNA) TE-buffer (10 mM Trix-HCl, 1 mM EDTA, pH 7.05) was added to a final volume of 440 ul and mixed together with 60 ul 2 M CaCl₂. Then the same amount of 2x TBS (Hepes 50 mM, NaCl 280 mM, Na₂HPO₄ 1.5 mM, pH 7.05) was added and mixed well. The precipitation solution was incubated for 30 minutes at 37°C and added directly to the cells which should be transfected.

### Example 8

### Culturing of Transfected Cells to Secrete Protein

The selected cells are treated for further cultivation in normal growth medium as described in section 8.

### Example 9

### F) Preparation of the Adjuvant of Purified Particles

To the desired concentration of antigen particles suspended in sterile saline, 1 : 10,000 volume Thimerosol, 1/10 volume of filter-sterilised 0.2 M Al K(SO4)₂ : 12 H₂0 were added. The pH was adjusted to 5.0 with sterile 1 N NaOH and the suspension was stirred at room temperature for 3 hours. The alum-precipitated antigen was recovered by centrifugation for 10 minutes at 2,000 rpm, resuspended in sterile normal saline containing 1;10,000 Thimerosol and aliquoted under sterile conditions.

### Example 10

Tables III - X give some of the results of ELISA analysis of immunogenic particles of the present invention as described below:
- Table III:: shows the ELISA data of the purified HBs antigen particle produced from any HBV sequence construct of the present invention including the pre-S₁ region with total deletion of pre-S₂ and deletions upstream of the pre-S₂ ATG and the S region with deletion of the S ATG and downstream the S ATG through the XBaI site (e.g. the construct of Fig. I-1) with the anti-pre-S₁ monoclonal antibody MA 18/7. The fractions 9-15 (Fig. XI) were pooled after CsCl sedimentation.
- Table IV:: shows the ELISA data of the purified HBS antigen particle produced from any HBV sequence construct of the present invention including the pre-S₁ region with total deletion of pre-S₂ and deletions upstream of the pre-S₂ ATG and the S region with deletion of the S ATG and downstream the S ATG through the XBaI site (e.g., the construct of Fig. I-1) with the anti-pre-S₂ monoclonal antibody MQ 19/10. The fractions 9-15 (Fig. XI) were pooled after CsCl sedimentation.
- Table V:: shows the ELISA data of the purified HBs antigen particle produced from an HBV sequence construct of the present invention including the pre-S₂ region with none of the pre-S₁ region and deletions upstream of the S ATG and downstream of the S ATG through the XBaI site, and the S region with deletion of the S ATG (e.g. the construct of Fig. II-1) with the anti-pre-S₁ monoclonal antibody MA 18/7. The fractions 9-15 (Fig. XII) were pooled after CsCl sedimentation.
- Table VI:: shows the ELISA data of the purified HBS antigen particle produced from an HBV sequence construct of the present invention including the pre-S₂ region with none of the pre-S₁ region and deletions upstream of the S ATG and downstream of the S ATG through the XBaI site, and the S region with deletion of the S ATG (e.g. the construct of Fig. II-1) with the anti-pre-S₂ monoclonal antibody MQ 19/10. The fractions 9-15 (Fig. XII) were pooled after CsCl sedimentation.

**Table III**

| | |
|---|---|
| CsCl-gradient | ELISA Measurement |
| | Monoclonal Antibody MA 18/7 |
| Fraction No. 9-15 (pooled) | E₄₉₂ = 0.839 |

**Table IV**

| | |
|---|---|
| CsCl-gradient | ELISA Measurement |
| | Monoclonal Antibody MQ 19/10 |
| Fraction No. 9-15 (pooled) | E₄₉₂ = 0.000 |

**Table V**

| | |
|---|---|
| CsCl-gradient | ELISA Measurement |
| | Monoclonal Antibody MA 18/7 |
| Fraction No. 9-15 (pooled) | E₄₉₂ = 0.000 |

**Table VI**

| | |
|---|---|
| CsCl-gradient | ELISA Measurement |
| | Monoclonal Antibody MQ 19/10 |
| Fraction No. 9-15 (pooled) | E₄₉₂ = 1.028 |

- Table VII:: shows the ELISA data of the purified HBs antigen particle produced from any HBV sequence construct of the present invention including the pre-S₁ region with total deletion of pre-S₂ and deletions upstream of the pre-S₂ ATG and the S region with deletion of the S ATG (e.g., the construct of Fig. VI-2) with the anti-pre-S₁ monoclonal antibody MA 18/7. The fractions 9-15 (Fig. XI) were pooled after CsCl sedimentation.
- Table VIII:: shows the ELISA data of the purified HBs antigen particle produced from any HBV sequence construct of the present invention including the pre-S₁ region with deletions upstream of the pre-S₂ ATG with deletion of the S ATG (e.g., the construct of Fig. VI-4) with the anti-pre-S₂ monoclonal antibody MQ 19/10. The fractions 9-15 (Fig. XI) were pooled after CsCl sedimentation.
- Table IX:: shows the ELISA data of the purified HBs antigen particle produced from an HBV sequence construct of the present invention including the pre-S₂ region with none of the pre-S₁ region and deletions upstream of the S ATG and the S region with deletion of the S ATG (e.g., the construct of Fig. VII-2) with the anti-pre-S₁ monoclonal antibody MA 18/7. The fractions 9-15 (Fig. XII) were pooled after CsCl sedimentation.
- Table X:: shows the ELISA data of the purified HBs antigen particle produced from an HBV sequence construct of the present invention including the pre-S₂ region with deletions upstream of the S ATG with deletion of the S ATG (e.g., the construct of Fig. VII-4) with the anti-pre-S₂ monoclonal antibody MQ 19/10. The fractions 9-15 (Fig. XII) were pooled after CsCl sedimentation.

**Table VII**

| | |
|---|---|
| CsCl-gradient | ELISA Measurement |
| | Monoclonal Antibody MA 18/7 |
| Fraction No. 9-15 (pooled) | E₄₉₂ = 1.273 |

**Table VIII**

| | |
|---|---|
| CsCl-gradient | ELISA Measurement |
| | Monoclonal Antibody MQ 19/10 |
| Fraction No. 9-15 (pooled) | E₄₉₂ = 0.000 |

**Table IX**

| | |
|---|---|
| CsCl-gradient | ELISA Measurement |
| | Monoclonal Antibody MA 18/7 |
| Fraction No. 9-15 (pooled) | E₄₉₂ = 0.000 |

**Table X**

| | |
|---|---|
| CsCl-gradient | ELISA Measurement |
| | Monoclonal Antibody MQ 19/10 |
| Fraction No. 9-15 (pooled) | E₄₉₂ = 0.985 |

- Table XI: shows the ELISA data of purified HBs antigen particles produced by construct including the entire pre-S₁ - pre-S₂ - S region under control of the LTR region of rous sarcoma virus after stimulation with stimulating substances (e.g. PMA) and the additional cotransfection with S (Fig. XIII).

**Table XI**

| | |
|---|---|
| CsCl-gradient | ELISA Measurement |
| | Monoclonal Antibody MA 18/7 |
| Fraction No. 9-15 (pooled) | E₄₉₂ = 0.125 |

- Figure XIV: shows the characterisation of the particles derived from gene constructs according to table III (Fig. I-1) and table V (Fig. II-1) cotransfected in C127 after purification in the CsCl gradient. The fraction collected had a smaller volume.
- Table XII: shows the serotyping of particles according to Fig. I-1 having the S sequence done in the Pettenkofer Institute.

**Table XII**

| |
|---|
| Results: |
| adw / ayw : positive |

From the foregoing, it will be obvious to those skilled in the art that various modifications in the above-described compositions and methods can be made without departing from the spirit and scope of the invention. Accordingly, the invention may be embodied in other specific forms without departing from the spirit or essential characteristics thereof. Present embodiments, therefore, are to be considered in all respects as illustrative and not restrictive, the scope of the invention being indicated by the appended claims rather than by the foregoing description, and all changes which come within the meaning and range of equivalency of the claims are therefore intended to be embraced therein.

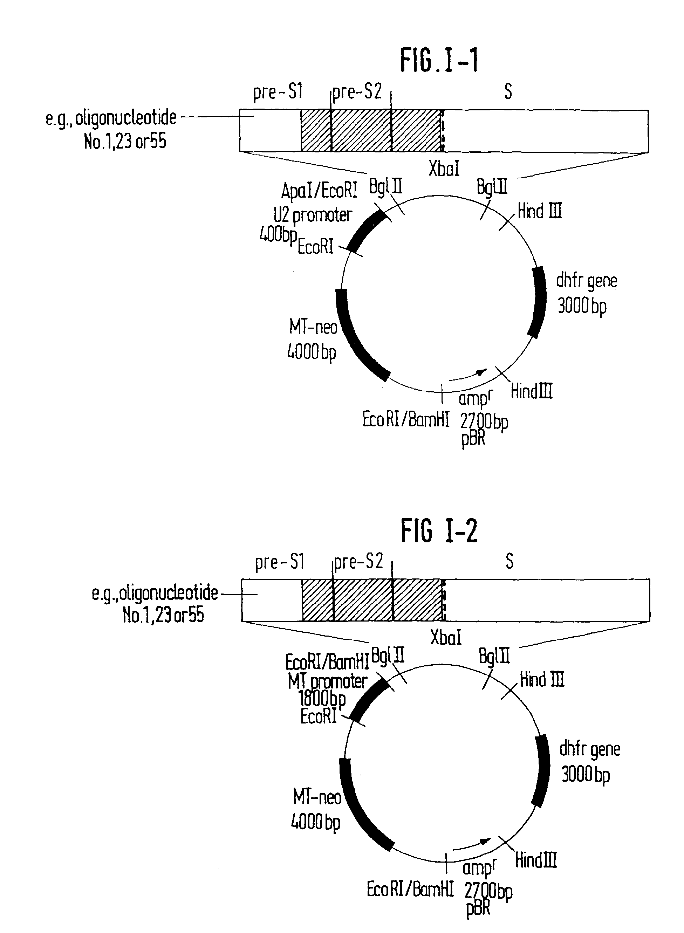

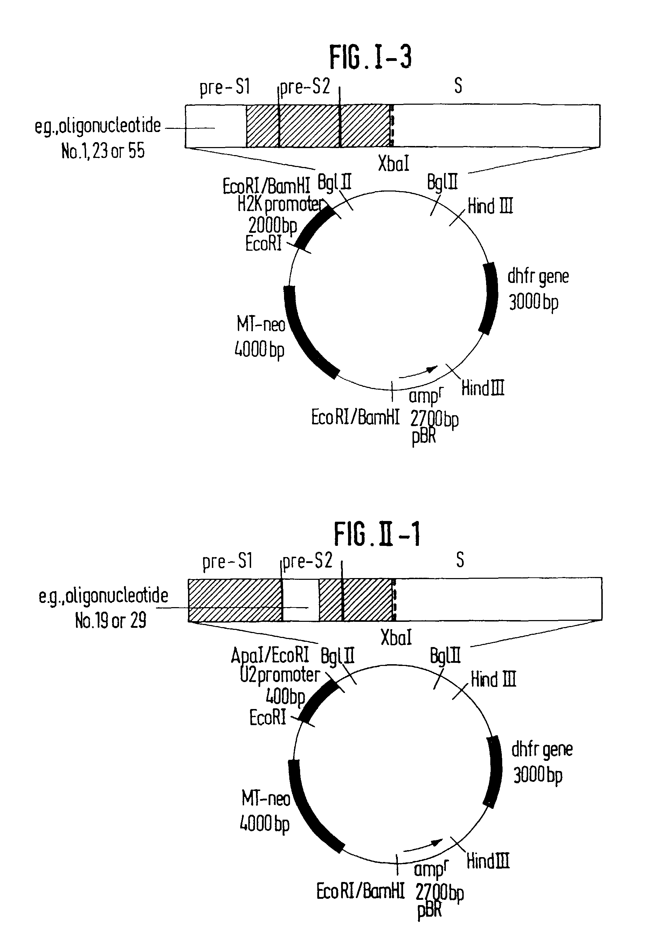

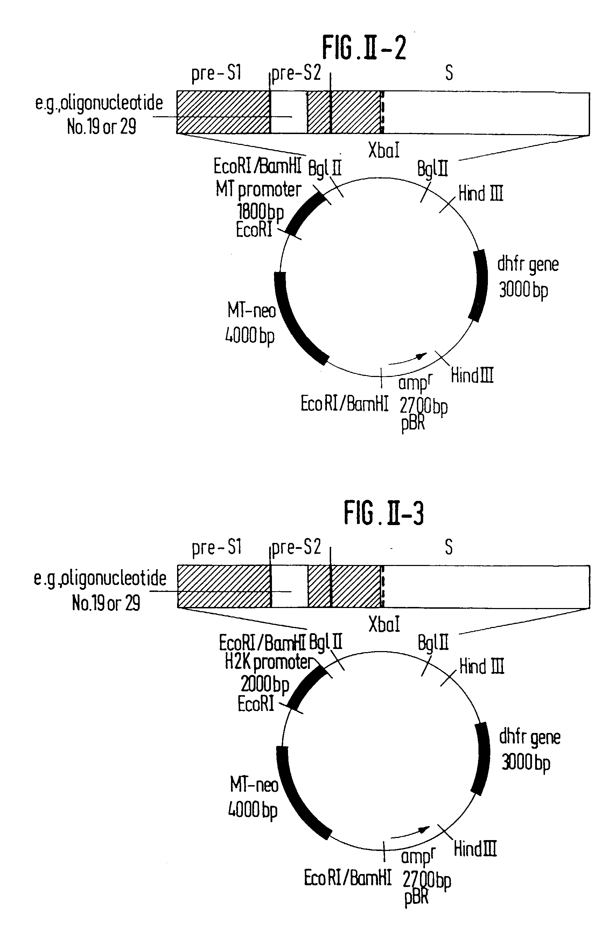

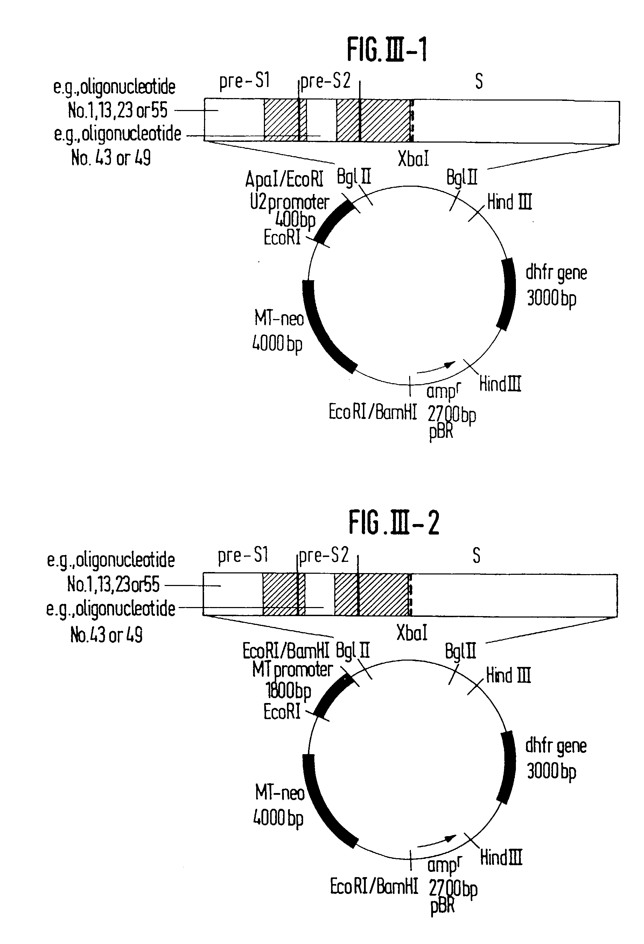

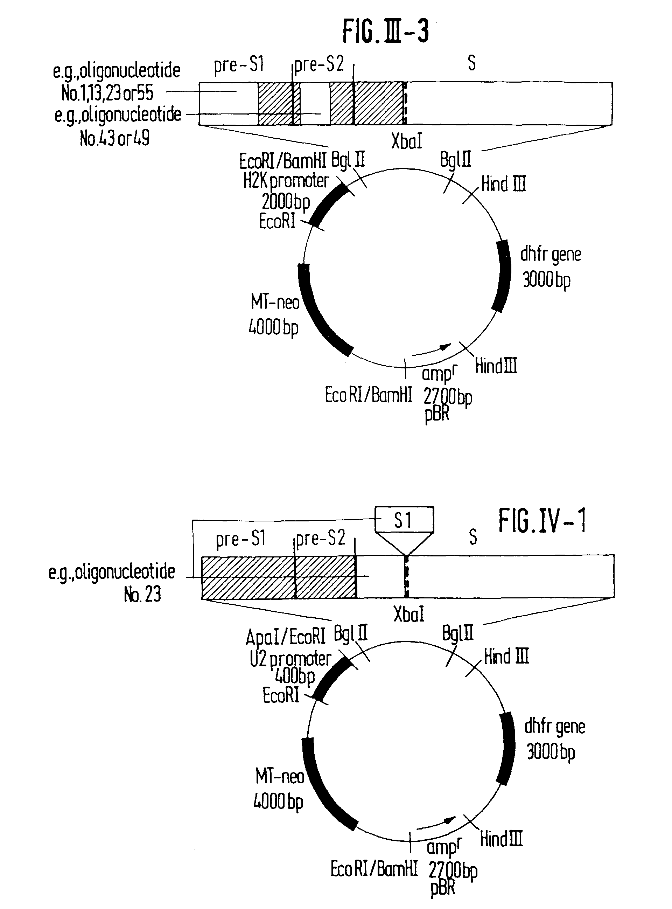

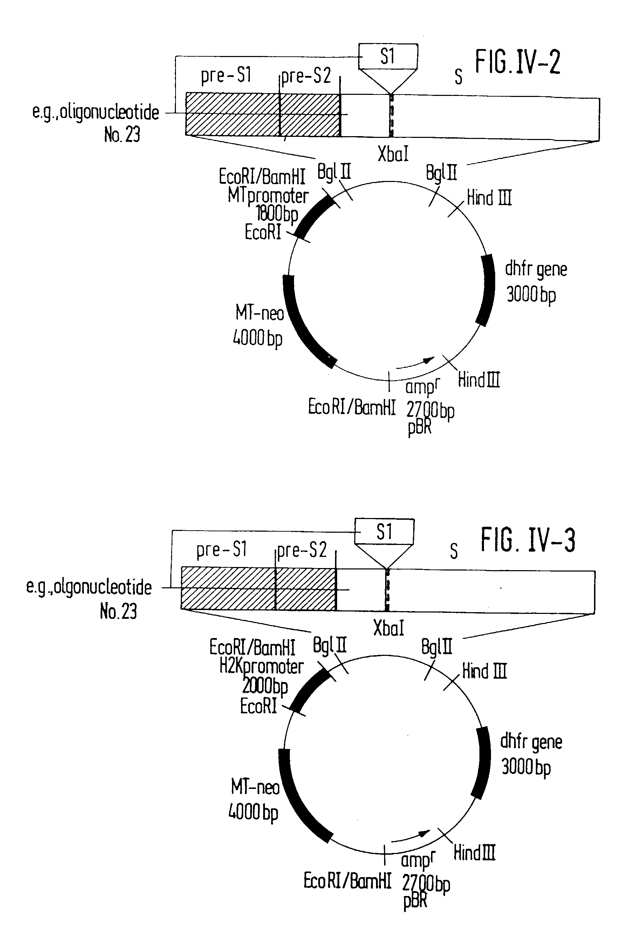

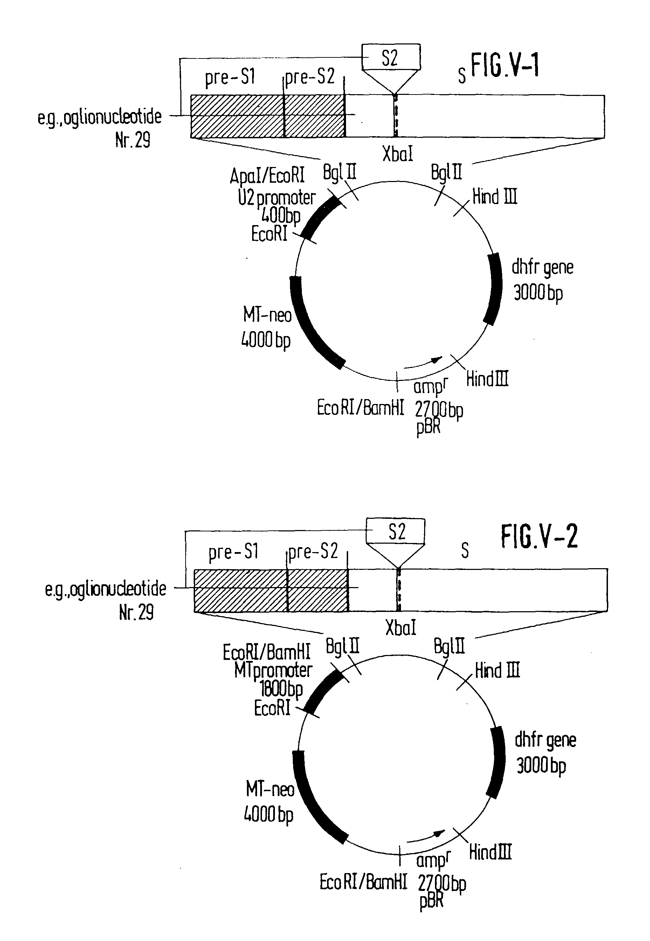

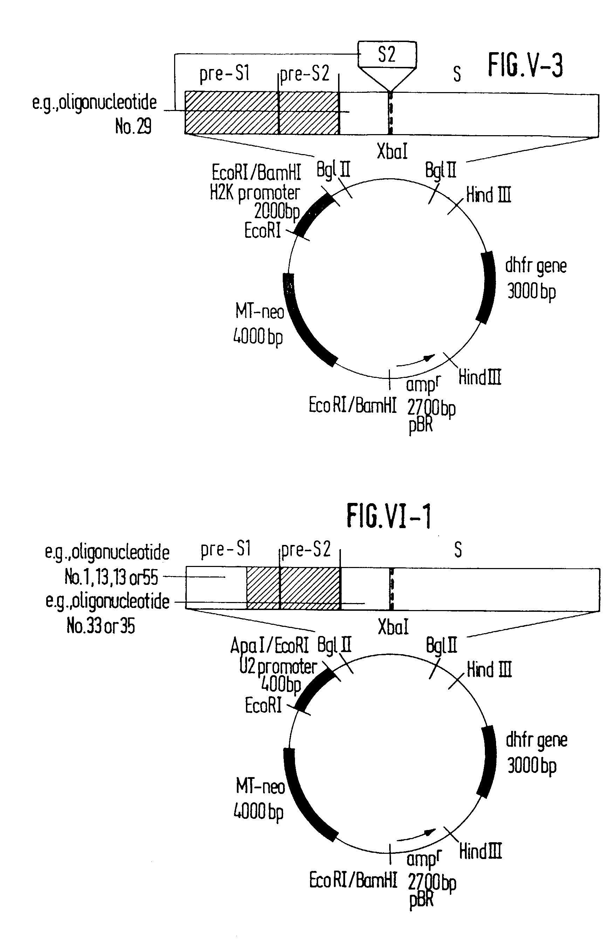

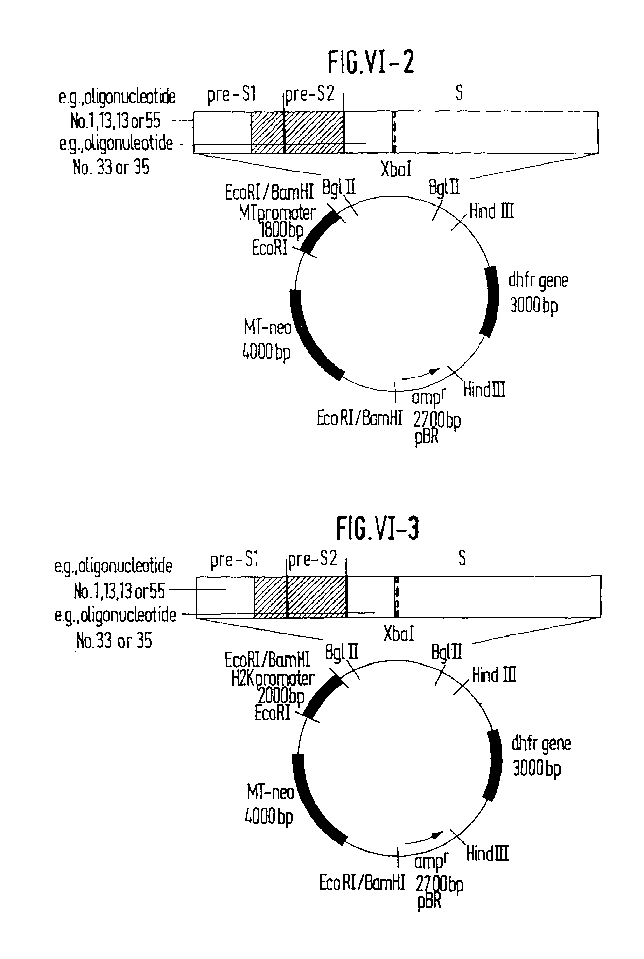

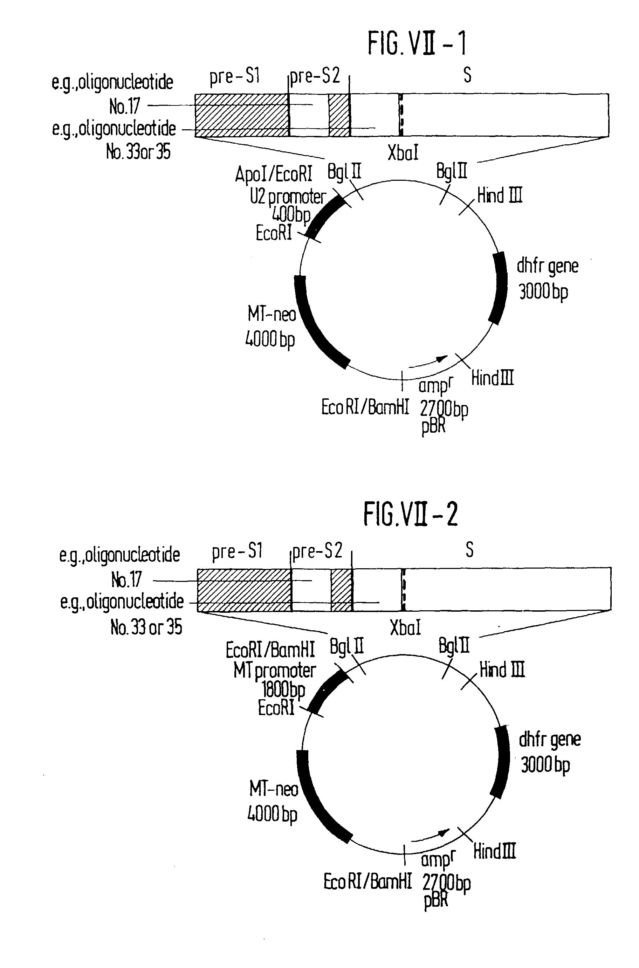

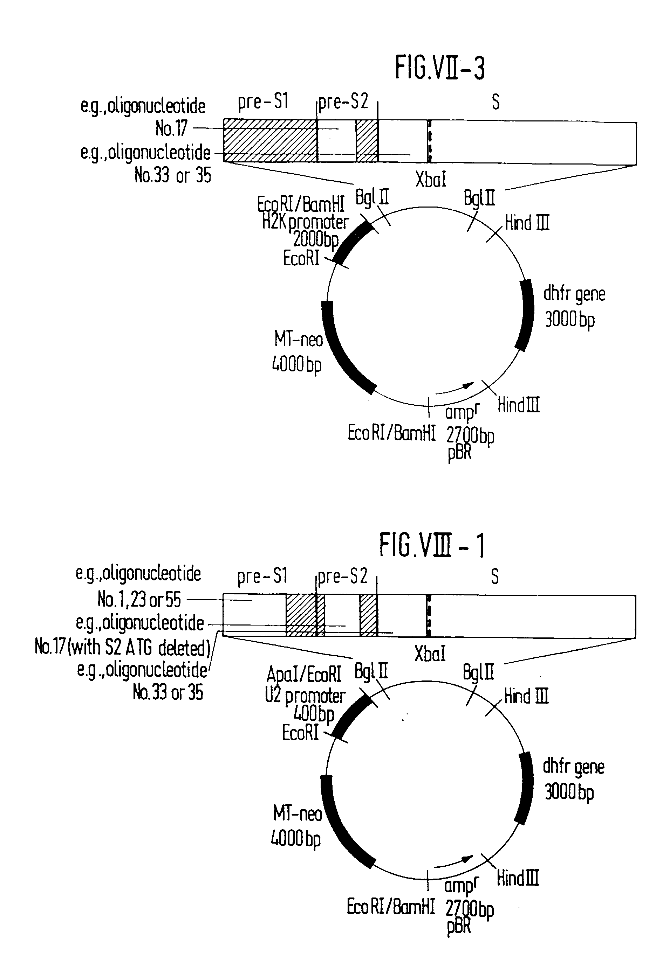

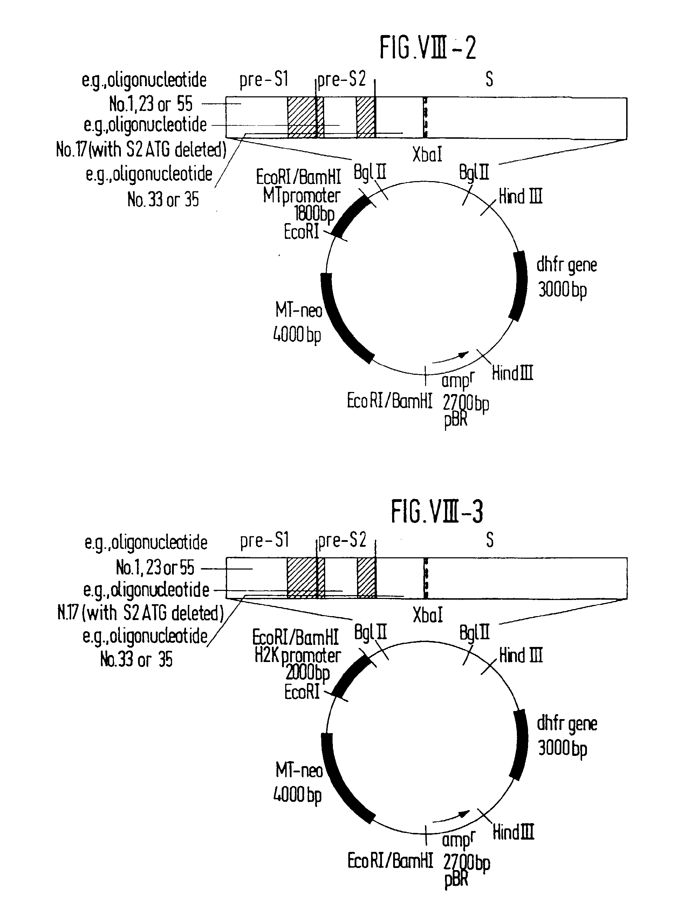

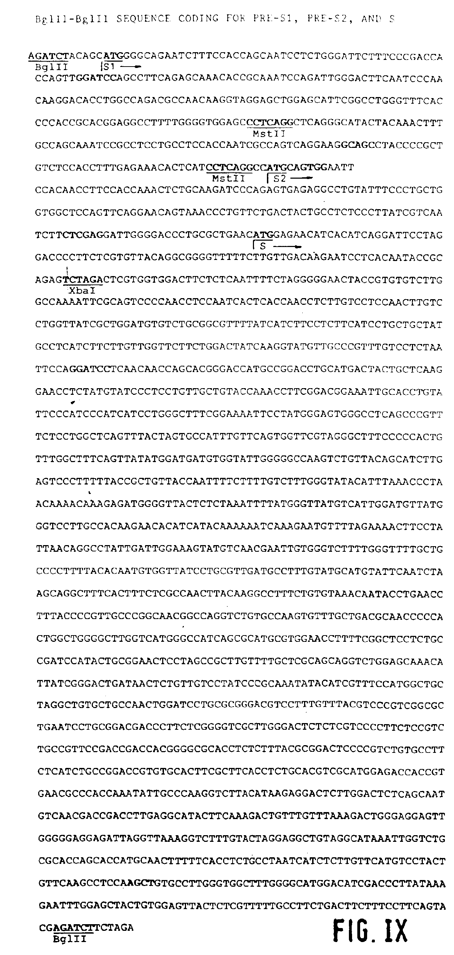

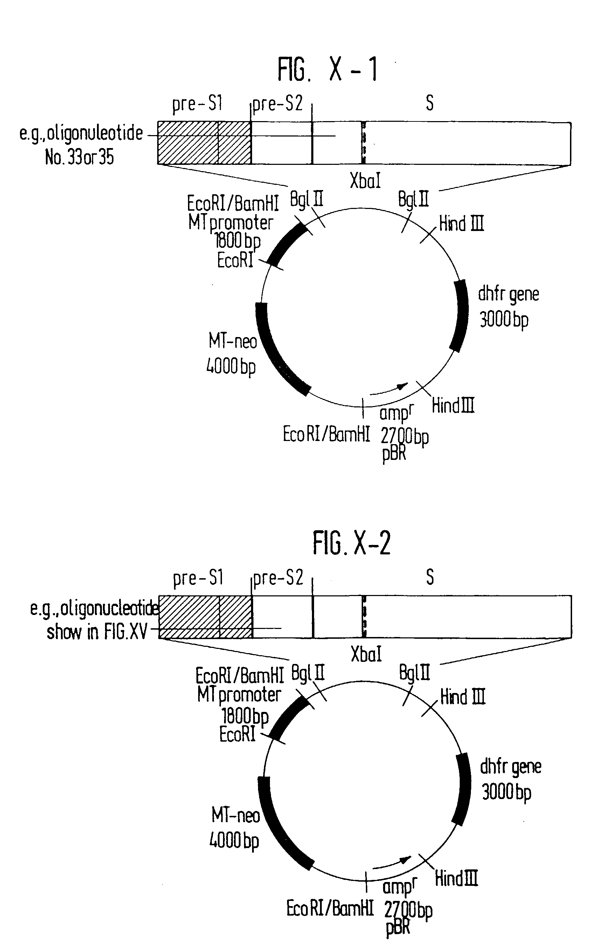

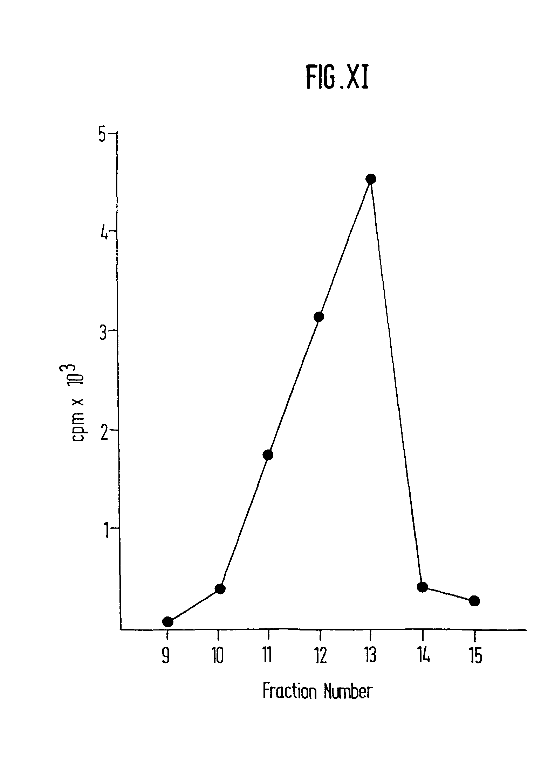

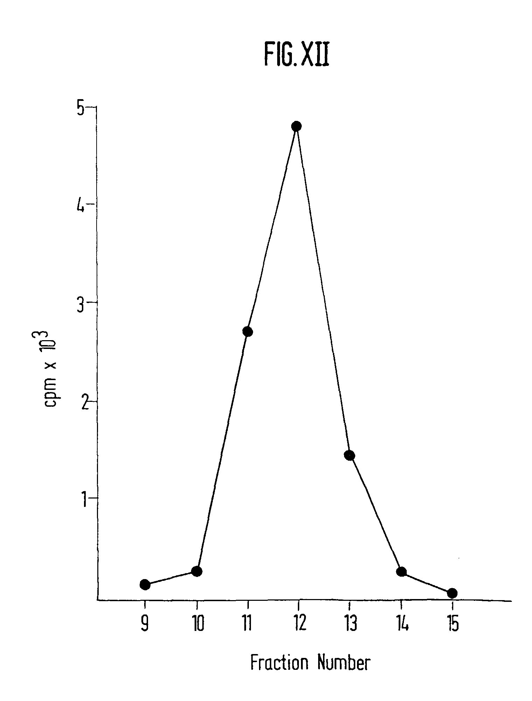

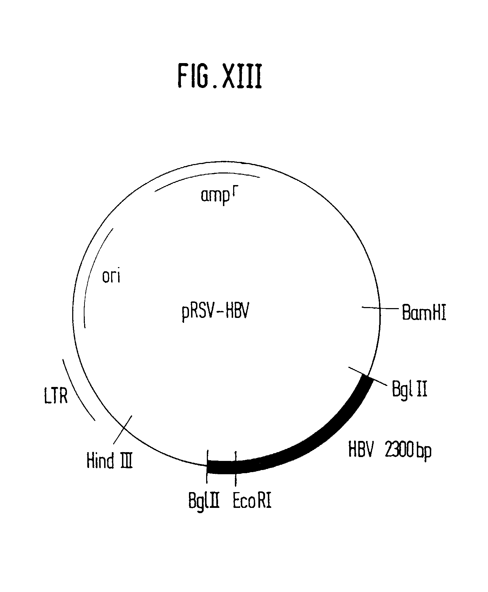

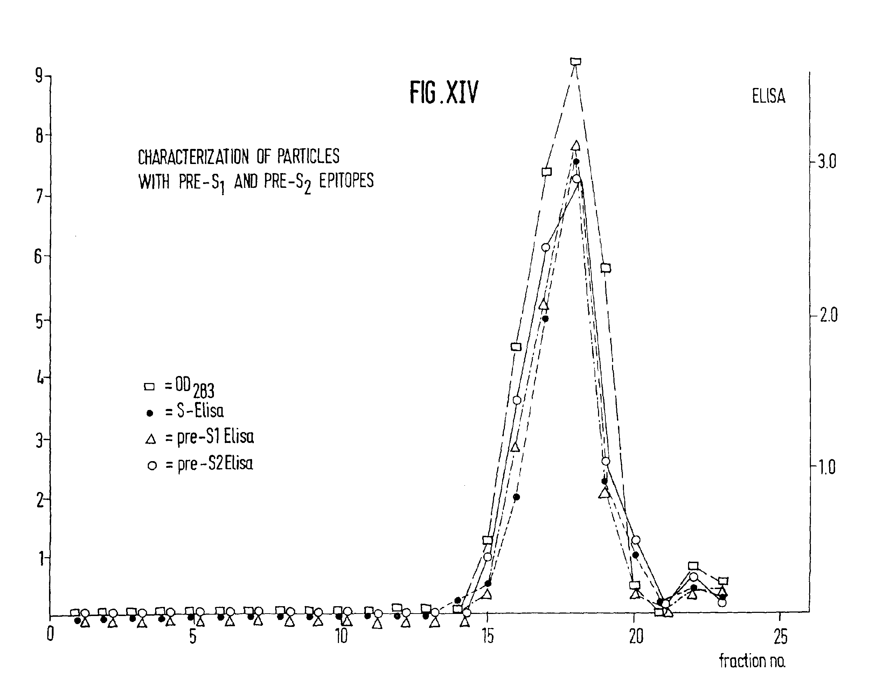

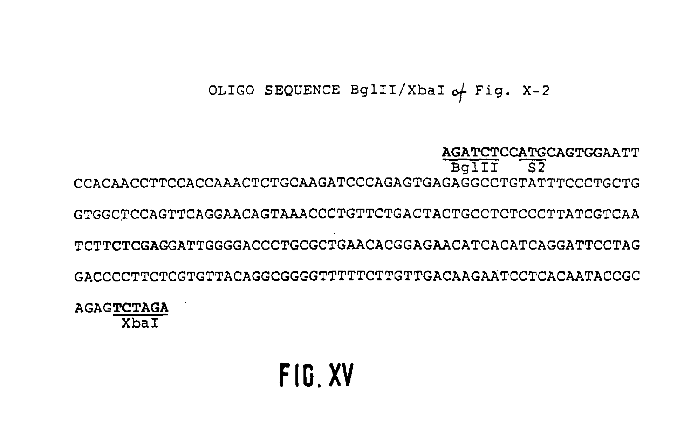

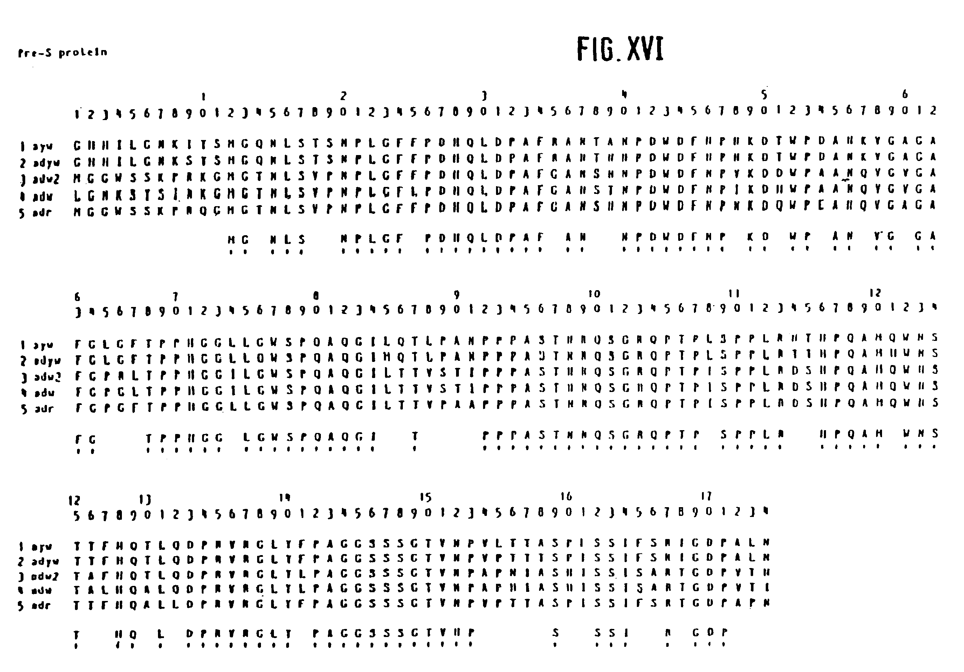

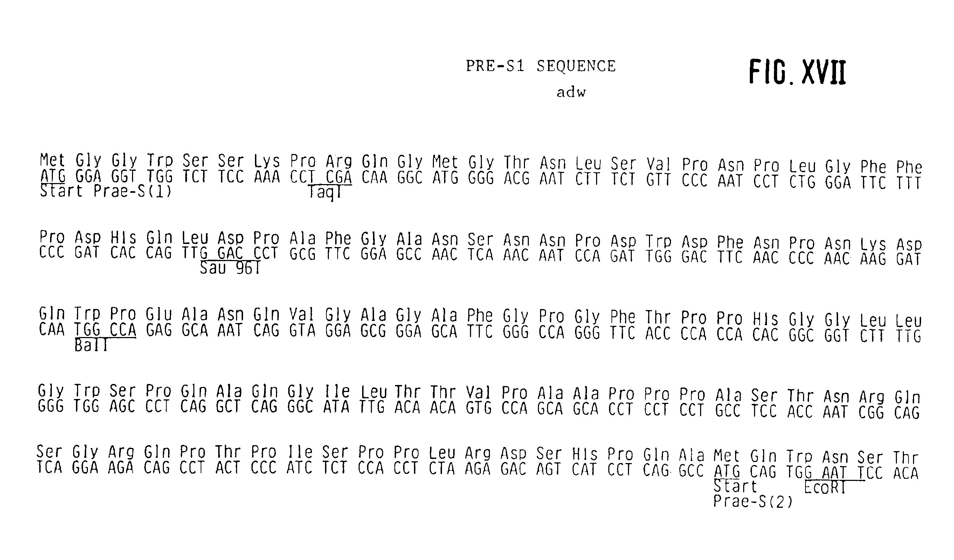

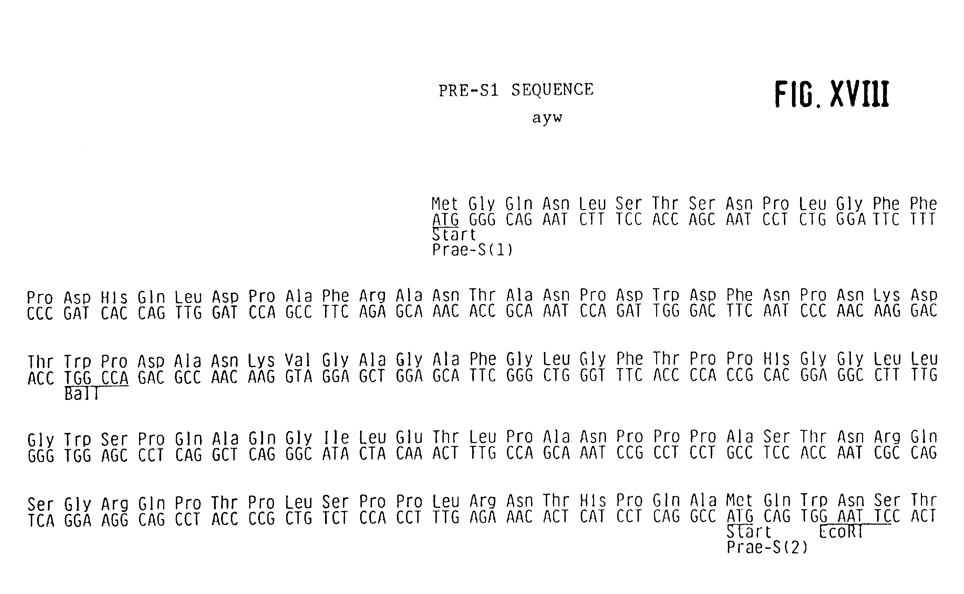

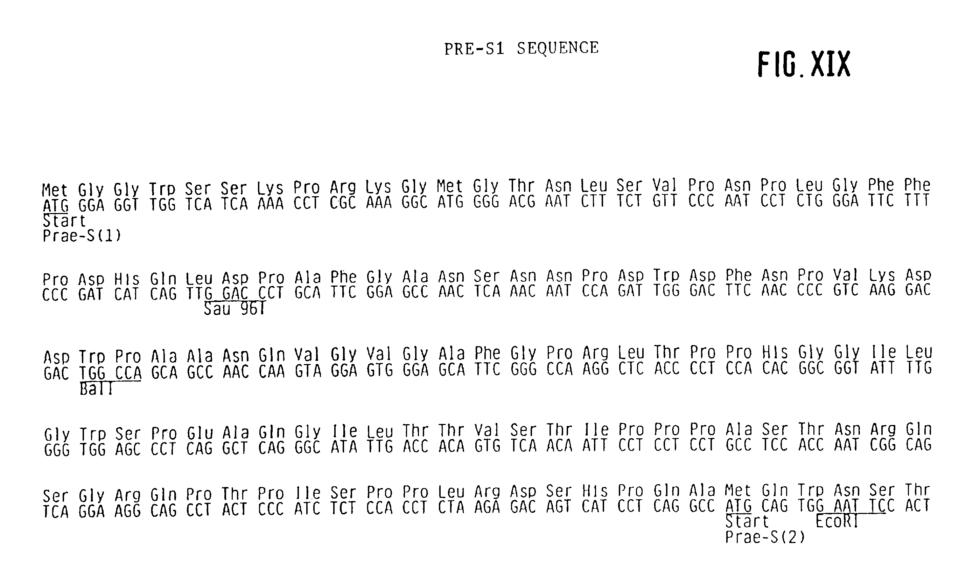

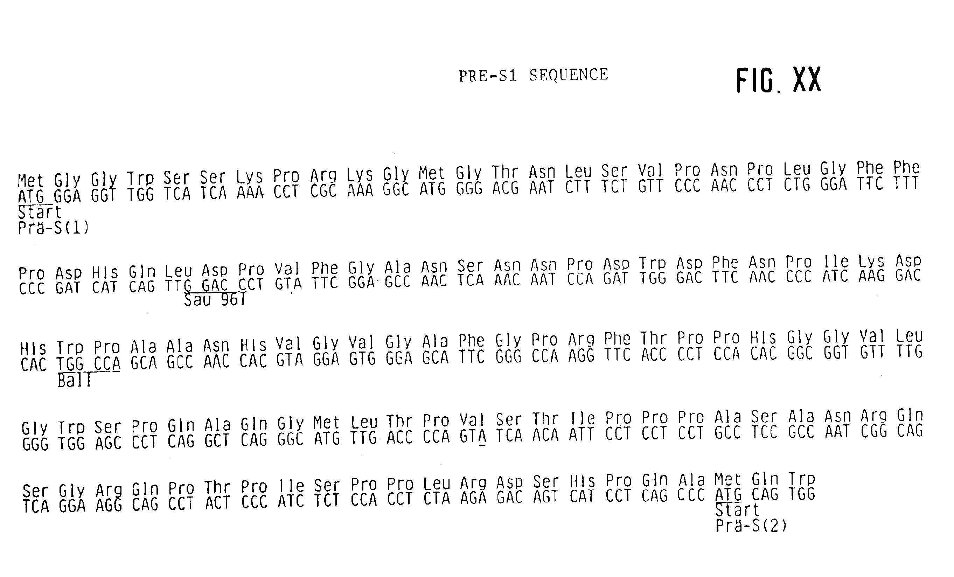

## Claims

1. An immunogenic particle comprising a plurality of copies of a first peptide monomer, said peptide monomer comprising a first discrete region and a second discrete region, said first region comprising an HBV pre-S₁ epitope and said second region comprising a peptide which upon secretion will direct formation of particles that will provoke an immune response against the pre-S₁ epitope, wherein said first peptide monomer constitutes more than five percent of all the protein in the particle and said particle is free of pre-S₁ peptide.

2. A particle of claim 1 wherein said second region comprises amino acid sequence of HBV S peptide or HBV core antigen.

3. A particle of claim 2 wherein said second region comprises amino acid sequence of the HBV S peptide.

4. A particle of claim 3 wherein said second region comprises all of the amino acid sequence of the HBV S peptide.

5. A particle of any one of the preceding claims wherein said peptide monomer is encoded by a recombinant DNA molecule comprising a first DNA sequence and a second DNA sequence, said first DNA sequence encoding expression of said first region and said second DNA sequence encoding expression of said second region, said first DNA sequence and second DNA sequence being in the same reading frame and being operatively linked to an expression control sequence in said recombinant DNA molecule.

6. A particle of claim 5 wherein said first DNA sequence comprises a nucleotide sequence corresponding to the nucleotide sequence of the HBV pre-S₁ region wherein the sequences flanking the pre-S₁ ATG have been changed from the natural sequence.

7. A particle of claim 5 wherein said first DNA sequence comprises a nucleotide sequence corresponding to the nucleotide sequence of the HBV pre-S₁ region wherein the 5'-terminus of the pre-S₁ region has been deleted.

8. A particle of claim 5 wherein said first DNA sequence comprises a nucleotide sequence corresponding to the nucleotide sequence of the HBV pre-S₁ region wherein the 3'-terminus of the pre-S₁ region has been deleted.

9. A particle of claim 5, wherein said first DNA sequence comprises the nucleotide sequence of part of the pre-S₁ coding region wherein a 5'-terminal region containing the pre-S₁ region ATG start codon has been replaced by a 5'-terminal region of the S coding region containing the S region ATG start codon, and said second DNA sequence comprises nucleotide sequence of the S coding region wherein the ATG start codon is absent.

10. A particle of claim 9, wherein the nucleotide sequence of said 5'-terminal region of the S region is ATGGAGAAC.

11. A particle of claim 10, wherein the nucleotide sequence of said 5'-terminal region of the S region is AACATGGAGAAC.

12. A particle of any one of claims 5 to 11 wherein said recombinant DNA molecule contains at least one nucleotide sequence of an oligonucleotide in Table I.

13. A particle of claim 12 wherein said first DNA sequence comprises the sequence of oligo no. 55 from Table I.

14. A particle of claim 12 wherein said first DNA sequence comprises the sequence of oligo no. 23 from Table I.

15. A particle of claim 13 or 14 wherein said second DNA sequence comprises the S coding sequence of the XbaI-Bg1II fragment of the HBV S gene shown in Figure IX.

16. A particle of claim 5, wherein said recombinant DNA molecule comprises HBV sequences described by Figure I-1, I-2, IV-1, IV-2, IV-3, VI-1, VI-2 or VI-3.

17. A particle of any one of the preceding claims wherein said first region is located closer to the N-terminus than said second region.

18. A particle of any one of the preceding claims which is at least 10 nm in diameter.

19. A particle of any one the preceding claims further comprising a plurality of copies of a second peptide monomer, said copies of said first peptide monomer and of said second peptide monomer being bound together, said second peptide monomer comprising amino acid sequence of the HBV S peptide.

20. A particle of claim 19 wherein said second peptide monomer comprises all of the amino acid sequence of the HBV S peptide.

21. A pharmaceutical preparation comprising an immunogenic particle of any one of the preceding claims in a sufficient concentration to elicit an immune response upon administration of said preparation, and a suitable carrier.

22. A preparation useful for production of antibodies, said preparation comprising an immunogenic particle of any one of claims 1 to 20 in sufficient concentration to cause production of antibodies upon administration of said preparation to an individual, and a suitable carrier.

23. A method of producing an immunogenic particle, said method comprising
preparing a recombinant DNA as defined in any one of claims 5 to 16,
transfecting a host cell with said recombinant DNA molecule,
culturing said host cell under conditions allowing expression and secretion of protein by said host cell, and
allowing under suitable conditions the aggregation of peptide monomers produced as a result of expression of exogenous DNA sequences within said host cell.

24. A method of producing an immunogenic particle of claim 23, said method further comprising transfecting said host cell with a DNA molecule encoding a peptide including the amino acid sequence of the IIBV S peptide.

25. A particle of any one of claims 1 to 20 for use in a method of vaccinating a human or animal against HBV.

26. Use of a particle of any one of claims 1 to 20 in the manufacture of a medicament for vaccinating a human or animal against HBV.
